# EUROPEAN PATENT SPECIFICATION

(11) **EP 3 818 144 B1**
(45) Date of publication and mention of the grant of the patent: **07.06.2023**
(21) Application number: 19734105.0
(22) Date of filing: 03.07.2019
(51) Int. Cl.: C12N 1/04, A61Q 19/00

(54) **AQUEOUS TOPICAL COMPOSITIONS COMPRISING VIABLE PROBIOTIC BACTERIA**
WÄSSRIGE TOPISCHE ZUSAMMENSETZUNGEN MIT LEBENSFÄHIGEN PROBIOTISCHEN BAKTERIEN
COMPOSITIONS TOPIQUES AQUEUSES COMPRENANT DES BACTÉRIES PROBIOTIQUES VIABLES

(30) Priority: 04.07.2018 EP 18181644
(43) Date of publication of application: 12.05.2021
(73) Proprietor: Chr. Hansen A/S, 2970 Hørsholm (DK)
(72) Inventor: DOLMER, Mogens, 2970 Hoersholm (DK); INGEMANN, Mette, 2970 Hoersholm (DK); WINNING, Mette, 2970 Hoersholm (DK)
(86) International application number: PCT/EP2019/067833
(87) International publication number: WO 2020/007906

(56) References cited:
- WO-A2-2013/188626
- ANONYMOUS: "Esse sensitive serum", INTERNET CITATION, 1 August 2016 (2016-08-01), XP002783641, Retrieved from the Internet: URL:https://www.esseskincare.com/esse_prod uct/sensitive-serum/ [retrieved on 2018-08-07]
- "Esse toner plus", INTERNET CITATION, 1 August 2016 (2016-08-01), XP002783654, Retrieved from the Internet: URL:https://www.esseskincare.com/esse_prod uct/toner-plus/ [retrieved on 2018-08-07]
- CORONADO ROBLES ET AL: "Probiotics promisingcosmetic ingredient or marketing tool?", INTERNET CITATION, 1 August 2016 (2016-08-01), XP002783655, Retrieved from the Internet: URL:https://www.researchgate.net/profile/M aria_Coronado/publication/308075735_Probio tics_-_promising_cosmetic_ingredient_or_ma rketing_tool/links/57d90bc508ae5f03b4993e0 d/Probiotics-promising-cosmetic-ingredient -or-marketing-tool.pdf [retrieved on 2018-08-07]
- Tetesept: "Proflora (R) Darm", , 1 October 2016 (2016-10-01), pages 1-5, XP055530134, Retrieved from the Internet: URL:https://www.tetesept.de/produkte/gesun dheit/erkaeltung/abwehrkraefte/proflora-da rm-27036.htm [retrieved on 2018-12-04]
- D Sharma: "Anti-Aging Effects of Probiotics", J Drugs Dermatol, 1 January 2016 (2016-01-01), pages 9-12, XP055498011, Retrieved from the Internet: URL:http://www.ncbi.nlm.nih.gov/pubmed/267 41377 [retrieved on 2018-08-07]

## Description

### Technical field of the invention

The present invention relates to aqueous topical compositions comprising at least 1·10⁶ CFU/g viable bacteria of at least one probiotic bacterial strain that remain viable in said composition for a period of time, such as up to 14 days or even longer at 25°C/60%RH or 30°C/75%RH, or 5°C. The present invention also relates to methods for preparing such aqueous topical compositions.

In particular, the present invention relates to a device that is suitable for long-term storage of viable bacteria of at least one probiotic bacterial strain and a water miscible solvent in separate compartments. At any time during storage time - until e.g. 3 years - the contents of the separate compartments comprising said viable bacteria of at least one probiotic bacterial strain and said water miscible solvent, respectively, can be combined to provide said aqueous topical compositions of the invention - ready to use.

The present invention also relates to methods for preparing said device and to the use of said delivery device for providing probiotic bacteria that remain viable in said aqueous topical composition.

### Background of the invention

It is well known in the art that probiotic bacteria in topical formulations do not survive in formulations that are moist (water activity between 0.40-0.90), comprise emulsifiers, preservatives and/or when the formulations are kept at high temperature.

Several manufactures have attempted to prepare topical formulations that comprise viable bacteria of at least one probiotic bacterial strain, but no commercial products provide viable bacteria of at least one probiotic bacterial strain in amounts of at least 1·10⁶ CFU/g that can be used by consumers to obtain the desired level of viable bacteria of at least one probiotic bacterial strain. Also, no aqueous topical products are available that can be stored for years at room temperature or even higher and still maintain viable bacteria of at least one probiotic bacterial strain at a very high level upon usage i.e. at least 1·10⁶ CFU/g. Topical formulations that are aqueous provide a pleasant sensory feeling upon application and thus often required by consumers.

Hence, there is a need in the art to provide aqueous topical formulations comprising viable bacteria of at least one probiotic bacterial strain that are stable after production, are viable after storage of up to three years at room temperature or after having been stored in a cool place such as in a refrigerator i.e. at about 25°C/60%RH, such as about 30°C/60%RH and such as about 5°C, and which are viable prior to application within assigned shelf life.

Hence, an improved topical formulation comprising viable bacteria of at least one probiotic bacterial strain would be advantageous, and in particular, a more stable topical formulation comprising viable bacteria of at least one probiotic bacterial strain for long term storage of viable bacteria of at least one probiotic bacterial strain would be advantageous.

### Summary of the invention

An object of the present invention relates to a stable aqueous topical composition comprising viable bacteria of at least one probiotic bacterial strain which maintains the desired high level of viable probiotic bacteria in the composition for a considerable amount of time.

In particular, it is an object of the present invention to provide a topical composition that solves the above-mentioned problems of the prior art with poor viability of the probiotic bacteria, poor stability of the probiotic bacteria in aqueous compositions and no long-term storage of the probiotic bacteria.

One aspect of the invention relates to a method of preparing an aqueous topical composition comprising
a) providing viable bacteria of at least one probiotic bacterial strain and optionally at least one buffer,
b) providing at least one water miscible solvent comprising at least one thickening agent to provide a mixture, and
c) adding said viable bacteria of at least one probiotic bacterial strain of step a) to said mixture of step b)
to prepare said aqueous topical composition having a pH of 3.0-8.0 and a viscosity of more than 2500 mPa·s and an amount of at least 1·10⁶ CFU/g of said viable bacteria of at least one probiotic bacterial strain.

Another aspect of the present invention relates to an aqueous topical composition comprising viable bacteria of at least one probiotic bacterial strain and at least one water miscible solvent, wherein an amount in the range of 1·10⁶-10¹¹ CFU/g of the viable bacteria of at least one probiotic bacterial strain is viable in the aqueous topical composition for a period of time, such as up to 14 days at 25°C/60%RH or 30°C/75%RH.

Yet another aspect of the present invention is to provide a probiotic bacteria delivery device comprising
- a first housing element comprising viable bacteria of at least one probiotic bacterial strain and optionally a desiccant, and
- a second housing element comprising at least one water miscible solvent,
wherein the amount of viable bacteria of at least one probiotic bacterial strain in said first housing element is at least 5·10⁶ CFU/g for up to 3 years at 25°C/60%RH or 30°C/75%RH.

Still another aspect of the present invention is to provide a method of preparing a delivery device according to the invention comprising the steps of
a) providing in a first housing element viable bacteria of at least one probiotic bacterial strain, and
b) providing in a second housing element at least one water miscible solvent
wherein a) and b) together provides said delivery device.

An even further aspect of the present invention relates to the use of a delivery device according to the invention for providing viable bacteria of at least one probiotic bacterial strain in an aqueous topical composition according to the invention for up to 14 days at 25°C/60%RH or 30°C/75%RH.

### Brief description of the figures

Figure 1a shows stability of *Lactobacillus rhamnosus* in an aqueous topical gel composition of the present invention.
Figure 1b shows stability of *Lactobacillus paracasei* in an aqueous topical gel composition of the present invention.
Figure 2a shows stability of *Bifidobacterium animalis* subsp *lactis* in an aqueous topical gel composition of the present invention.
Figure 2b shows stability of *Lactobacillus rhamnosus* in an aqueous topical lotion composition of the present invention.
Figure 3a shows stability of a *Lactobacillus rhamnosus* blend in the first housing element.
Figure 3b shows stability of a *Bifidobacterium animalis* subsp *lactis* blend in the first housing element.

### Detailed description of the invention

An aspect of the invention relates to a method of preparing an aqueous topical composition comprising
a) providing viable bacteria of at least one probiotic bacterial strain in an amount of at least 5·10⁶ CFU/g and at least one buffer,
b) providing a mixture comprising at least one thickening agent selected from one or more of carbomer, poloxamer, PEG/macrogol and/or carboxylic acid and at least 60%(w/w) water, and
c) adding said viable bacteria of at least one probiotic bacterial strain of step a) to said mixture of step b)
to prepare said aqueous topical composition having a pH of 4.0-6.7 and a viscosity of more than 2500 mPa·s and an amount of 1·10⁶-10¹¹ CFU/g of said viable bacteria of at least one probiotic bacterial strain, and wherein said viable bacteria of at least one probiotic bacterial strain are viable in the aqueous topical composition for up to 7 days at 25°C/60%RH.

In an embodiment a method of preparing an aqueous topical composition is disclosed comprising
a) providing viable bacteria of at least one probiotic bacterial strain in an amount of at least 5·10⁶ CFU/g and at least one buffer,
b) providing a mixture comprising at least one thickening agent selected from one or more of carbomer, poloxamer, PEG/macrogol and/or carboxylic acid and at least 60%(w/w) water, and
c) adding said viable bacteria of at least one probiotic bacterial strain of step a) to said mixture of step b)
to prepare said aqueous topical composition having a pH of 4.0-6.7 and a viscosity of more than 2500 mPa·s and an amount of 1·10⁶-10¹¹ CFU/g of said viable bacteria of at least one probiotic bacterial strain, and wherein said viable bacteria of at least one probiotic bacterial strain are viable in the aqueous topical composition for up to 7 days at 25°C/60%RH.

In an embodiment a method of preparing an aqueous topical composition is disclosed comprising
a) providing a dry composition of viable bacteria of at least one probiotic bacterial strain in an amount of at least 5·10⁶ CFU/g and at least one buffer, wherein said dry composition of viable bacteria of at least one probiotic bacterial strain is powdered having a water activity of no more than 0.30 and a particle size distribution below 500µm
b) providing a mixture comprising at least one thickening agent selected from one or more of poloxamers and at least 60%(w/w) water to provide said mixture having a viscosity of about 100 mPa·s when the temperature is no more than 10°C, and a viscosity of about 1000 mPa·s when the temperature is above 25°C, and
c) adding said dry composition of step a) to said mixture of step b)

to prepare said aqueous topical composition having a viscosity of less than 500 mPa·s when the temperature is no more than 10°C, and a viscosity of more than 2500 mPa·s when the temperature is above 30°C, and wherein said aqueous topical composition comprises an amount in the range of 1·10⁶-10¹¹ CFU/g of said viable bacteria of at least one probiotic bacterial strain and,
wherein said viable bacteria of at least one probiotic bacterial strain are viable in the aqueous topical composition for up to 7 days at 25°C/60%RH.

In an aspect of the invention an aqueous topical composition is disclosed comprising at least 60 %(w/w) water and
- viable bacteria of at least one probiotic bacterial strain,
wherein an amount of 1·10⁶-10¹¹ CFU/g of said viable bacteria of at least one probiotic bacterial strain is viable in the aqueous topical composition for up to 7 days at 25°C/60%RH, and wherein said composition comprises at least one thickening agent selected from one or more of carbomer, poloxamer, PEG/macrogol and/or carboxylic acid, and at least one buffer.

In yet another aspect a probiotic bacteria delivery device is disclosed comprising
- a first housing element comprising viable bacteria of at least one probiotic bacterial strain and at least one buffer and optionally a desiccant, and
- a second housing element comprising at least one thickening agent selected from one or more of carbomer, poloxamer, PEG/macrogol and/or carboxylic acid, and at least 60% (w/w) water, and at least one buffer,
wherein the amount of viable bacteria of said at least one probiotic bacterial strain in said first housing element is at least 5·10⁶ CFU/g for up to 3 years at 25°C/60%RH.

In another aspect, a method of preparing a delivery device is disclosed comprising the steps of
a) providing in a first housing element viable bacteria of at least one probiotic bacterial strain in an amount of at least 5·10⁶ CFU/g for up to 3 years at 25°C/60%RH, and
b) providing in a second housing element at least one thickening agent selected from one or more of carbomer, poloxamer, PEG/macrogol and/or carboxylic acid and at least 60% (w/w) water, and at least one buffer
wherein a) and b) together provides said delivery device.

In another aspect, the use of a delivery device is disclosed for providing viable bacteria of at least one probiotic bacterial strain in an aqueous topical composition according to the invention for at for at least 7 days at 25°C/60%RH or 30°C/75%RH.

One aspect of the invention relates to a method of preparing an aqueous topical composition comprising
a) providing viable bacteria of at least one probiotic bacterial strain and optionally at least one buffer,
b) providing at least one water miscible solvent comprising at least one thickening agent to provide a mixture, and
c) adding said viable bacteria of at least one probiotic bacterial strain of step a) to said mixture of step b)
to prepare said aqueous topical composition having a pH of 3.0-8.0 and a viscosity of more than 2500 mPa·s, such as more than 2700 mPa·s, such as more than 3000 mPa·s, such as more than 3300 mPa·s, such as more than 3600 mPa·s, such as more than 3900 mPa·s, such as more than 5000 mPa·s, such as more than 6000 mPa·s, such as more than 7500 mPa·s, such as more than 10000 mPa·s and an amount in the range of 1·10⁶-10¹¹ CFU/g, such as at least 10⁶ CFU/, such as at least 10⁷ CFU/g, such as at least 10⁸ CFU/g, such as at least 10⁹, such as at least 10¹⁰, such as at least 10¹¹ CFU/g of said viable bacteria of at least one probiotic bacterial strain. In an embodiment of the invention, the pH may be 3-7.5, such as 3-7.0, such as 3.5-7.0, such as 4.0-6.7, such as 4.0-7.0, such as 4.0-8.0, such as 4.5-7.0, such as 4.5-6.5, such as 3-4, such as 3.5-4.5, such as 4.5-5.5, such as more than 3, such as more than 3.5, such as more than 4.0, such as more than 5.0, such as more than 5.5. In a preferred embodiment, the pH is 4.0-7.0.

An embodiment of the invention relates to a method of preparing an aqueous topical composition according to the invention, wherein said mixture in step b) has a viscosity of less than 2500 mPa·s, such as less than 2200 mPa·s, such as less than 2000 mPa·s, such as less than 1800 mPa·s, such as less than 1500 mPa·s, such as less than 1000 mPa·s, such as less than 750 mPa·s, such as less than 500 mPa·s.

Typically, a consumer will store the aqueous topical composition according to the invention at room temperature or in the refrigerator of their home in their country of origin. Common room temperature ranges vary quite a lot depending on country, time of day etc. In a preferred embodiment, the temperature of the aqueous topical composition of the invention or the delivery device comprising the aqueous topical composition of the invention may be in the range of from about 18°C-32°C, such as 20°C-32°C, such as 22°C-32°C, such as 24°C-31°C, such as 25°C-30°C, such as 20°C-30°C, such as 22°C-30°C, such as 23°C-28°C, such as 24°C-26°C and preferably 25°C or 30°C. Also, temperatures in refrigerators vary quite a lot and may comprise from about 1°C-15°C, such as 1°C-10°C, such as 2°C-9°C, such as 3°C-8°C, such as 4°C-6°C, and preferably 5°C according to an embodiment of the invention.

An embodiment of the invention relates to a method of preparing an aqueous topical composition according to the invention, wherein said viable bacteria of at least one probiotic bacterial strain are viable in said aqueous topical composition for at least 5 minutes at 25°C/60%RH or 30°C/75%RH or 5°C, such as for at least 20 minutes at 25°C/60%RH or 30°C/75%RH or 5°C, such as at least 1 hour at 25°C/60%RH or 30°C/75%RH or 5°C, such as at least 4 hours at 25°C/60%RH or 30°C/75%RH or 5°C, such as at least 12 hours at 25°C/60%RH or 30°C/75%RH or 5°C, such as at least 24 hours at 25°C/60%RH or 30°C/75%RH or 5°C, such as at least 2 days at 25°C/60%RH or 30°C/75%RH or 5°C, such as at least 3 days at 25°C/60%RH or 30°C/75%RH or 5°C, such as at least 4 days at 25°C/60%RH or 30°C/75%RH or 5°C, such as at least 5 days at 25°C/60%RH or 30°C/75%RH or 5°C, such as at least 6 days at 25°C/60%RH or 30°C/75%RH or 5°C, such as at least 7 days at 25°C/60%RH or 30°C/75%RH or 5°C, such as at least 8 days at 25°C/60%RH or 30°C/75%RH or 5°C, such as at least 9 days at 25°C/60%RH or 30°C/75%RH or 5°C, such as at least 10 days at 25°C/60%RH or 30°C/75%RH or 5°C, such as at least 14 days at 25°C/60%RH or 30°C/75%RH or 5°C.

Another embodiment of the invention relates to a method of preparing an aqueous topical composition according to the invention, wherein said viable bacteria of at least one probiotic bacterial strain are viable in said aqueous topical composition for up to 5 minutes at 25°C/60%RH or 30°C/75%RH or 5°C, such as for up to 20 minutes at 25°C/60%RH or 30°C/75%RH or 5°C, such as up to 1 hour at 25°C/60%RH or 30°C/75%RH or 5°C, such as up to 4 hours at 25°C/60%RH or 30°C/75%RH or 5°C, such as up to 12 hours at 25°C/60%RH or 30°C/75%RH or 5°C, such as up to 24 hours at 25°C/60%RH or 30°C/75%RH or 5°C, such as up to 2 days at 25°C/60%RH or 30°C/75%RH or 5°C, such as up to 3 days at 25°C/60%RH or 30°C/75%RH or 5°C, such as up to 4 days at 25°C/60%RH or 30°C/75%RH or 5°C, such as up to 5 days at 25°C/60%RH or 30°C/75%RH or 5°C, such as up to 6 days at 25°C/60%RH or 30°C/75%RH or 5°C, such as up to 7 days at 25°C/60%RH or 30°C/75%RH or 5°C, such as up to 8 days at 25°C/60%RH or 30°C/75%RH or 5°C, such as up to 9 days at 25°C/60%RH or 30°C/75%RH or 5°C, such as up to 10 days at 25°C/60%RH or 30°C/75%RH or 5°C, such as up to 14 days at 25°C/60%RH or 30°C/75%RH or 5°C, such as up to 18 days at 25°C/60%RH or 30°C/75%RH or 5°C, such as up to 22 days at 25°C/60%RH or 30°C/75%RH or 5°C, such as up to 26 days at 25°C/60%RH or 30°C/75%RH or 5°C, such as up to 30 days at 25°C/60%RH or 30°C/75%RH or 5°C, such as up to 2 months at 25°C/60%RH or 30°C/75%RH or 5°C.

An even further embodiment of the invention relates to a method of preparing an aqueous topical composition according to the invention, wherein said viable bacteria of at least one probiotic bacterial strain is viable in said aqueous topical composition for 5 minutes to 2 months, such as 5 minutes to 1 month, such as 5 minutes to 14 days, such as 5 minutes to 7 days, such as 5 minutes to 4 days.

It has surprisingly been shown that said probiotic bacteria remain viable in said aqueous topical composition from the moment the composition is ready to use by the user/consumer for at least 5 minutes at 25°C/60%RH or 30°C/75%RH or 5°C or for up to 5 minutes at 25°C/60%RH or 30°C/75%RH or 5°C.

Additionally, and even more surprisingly, said probiotic bacteria remain viable in said aqueous topical composition for at least 7 days or even up to 14 days at 25°C/60%RH or 30°C/75%RH or 5°C. This provides the consumer with the advantage of a having a stable topical composition comprising viable bacteria of at least one probiotic bacterial strain ready to use for a long duration of time.

An embodiment of the invention relates to a method of preparing an aqueous topical composition according to the invention, wherein said viable bacteria of at least one probiotic bacterial strain is provided in a dry composition. Using a dry composition gives the advantage of having a stable product at room temperature for at least 1 month at 25°C/60%RH or 30°C/75%RH or 5°C, such as at least 3 months at 25°C/60%RH or 30°C/75%RH or 5°C, such as at least 6 months at 25°C/60%RH or 30°C/75%RH or 5°C, such as at least 9 months at 25°C/60%RH or 30°C/75%RH or 5°C, such as at least 12 months at 25°C/60%RH or 30°C/75%RH or 5°C, such as at least 18 months at 25°C/60%RH or 30°C/75%RH or 5°C, such as at least 24 months at 25°C/60%RH or 30°C/75%RH or 5°C, such as at least 30 months at 25°C/60%RH or 30°C/75%RH or 5°C, or even at least for 36 months at 25°C/60%RH or 30°C/75%RH or 5°C.

An embodiment of the invention relates to a method of preparing an aqueous topical composition according to the invention comprising
a) providing viable bacteria of at least one probiotic bacterial strain and at least one or more buffers,
b) providing at least one water miscible solvent comprising at least one thickening agent and one or more acids or one or more bases to provide a mixture, and optionally an emulsifier and one or more hydrophobic compound, and
c) adding said viable bacteria of at least one probiotic bacterial strain of step a) to said mixture of step b) and optionally further adding a buffer
to prepare said aqueous topical composition having a pH of 3.0-8.0 and a viscosity of more than 2500 mPa·s, such as more than 2700 mPa·s, such as more than 3000 mPa·s, such as more than 3300 mPa·s, such as more than 3600 mPa·s, such as more than 3900 mPa·s, such as more than 5000 mPa·s, such as more than 7500 mPa·s, such as more than 10000 mPa·s, and an amount in the range of 1·10⁶-10¹¹ CFU/g, such as at least 10⁶ CFU/, such as at least 10⁷ CFU/g, such as at least 10⁸ CFU/g, such as at least 10⁹ CFU/g, such as at least 10¹⁰ CFU/g, such as at least 10¹¹ CFU/g of said viable bacteria of at least one probiotic bacterial strain.

According to the invention, an acid or base is used to adjust the pH, whereas a buffer is used to keep the pH at a desired level. It has surprisingly been shown that by controlling the pH at a desired level by using an acid and/or base, the viscosity of the mixture in b) can be controlled when using carbomer thickening agents of the invention thus providing an aqueous topical composition of the invention having a pH of 3.0-8.0, such as preferably 4.0-7.0, and a viscosity of more than 2500 mPa·s when the viable bacteria of at least one probiotic bacterial strain and at least one or more buffers is mixed with the mixture of step b) of the invention.

In an embodiment of the invention, relating to a method of preparing an aqueous topical composition according to the invention, said viable bacteria of at least one probiotic bacterial strain is provided in a dry composition. In an embodiment, said dry composition has a particle size diameter of no more than 1000 µm, such as no more than 900 µm, such as no more than 800 µm, such as no more than 700 µm, such as no more than 600 µm, such as no more than 500 µm, such as no more than 400 µm, such as no more than 300 µm, such as no more than 200 µm, such as no more than 150 µm, such as no more than 100 µm. In a preferred embodiment, said dry composition has a particle size diameter of no more than 200 µm. In another embodiment, said dry composition may further comprise a thickening agent.

An embodiment of the invention relates to a method of preparing an aqueous topical composition according to the invention, wherein the composition of viable bacteria of at least one probiotic bacterial strain comprises at least one acid or base. Adding an acid or base will help to keep the composition at the desired pH level so that the thickening can be controlled. Preferably, when said viable bacteria of at least one probiotic bacterial strain comprises at least one acid or base, said viable bacteria of at least one probiotic bacterial strain is a dry composition.

An embodiment of the invention relates to method of preparing an aqueous topical composition according to the invention comprising
a) providing a dry composition of viable bacteria of at least one probiotic bacterial strain in an amount of at least 5·10⁶ CFU/g and at least one buffer,
b) providing at least one water miscible solvent comprising at least one thickening agent to provide a mixture having a pH of 2-3 and a viscosity of less than 2500 mPa·s, and
c) adding said dry composition of step a) to said mixture of step b) to prepare said aqueous topical composition having a pH of 3.0-8.0 and a viscosity of more than 2500 mPa·s and an amount in the range of 1·10⁶-10¹¹ CFU/g of said viable bacteria of at least one probiotic bacterial strain.

An embodiment of the invention relates to method of preparing an aqueous topical composition according to the invention comprising
a) providing a dry composition of viable bacteria of at least one probiotic bacterial strain in an amount of at least 5·10⁶ CFU/g and at least one buffer and at least one or more acid or base,
b) providing at least one water miscible solvent comprising at least one thickening agent to provide a mixture having a pH of 2-3 and a viscosity of less than 2500 mPa·s, and
c) adding said dry composition of step a) to said mixture of step b) to prepare said aqueous topical composition having a pH of 3.0-8.0 and a viscosity of more than 2500 mPa·s and an amount of at least 1·10⁶-10⁹ CFU/g of said viable bacteria of at least one probiotic bacterial strain.

An embodiment of the invention relates to method of preparing an aqueous topical composition according to the invention comprising
a) providing a dry composition of viable bacteria of at least one probiotic bacterial strain in an amount of at least 5·10⁶ CFU/g and at least one buffer,
b) providing at least one water miscible solvent comprising at least one thickening agent selected from one or more carbomers to provide a mixture having a pH of 2-3 and a viscosity of less than 2500 mPa·s, and
c) adding said dry composition of step a) to said mixture of step b) to prepare said aqueous topical composition having a pH of 3.0-8.0 and a viscosity of more than 2500 mPa·s and an amount in the range of 1·10⁶-10¹¹ CFU/g of said viable bacteria of at least one probiotic bacterial strain.

An embodiment of the invention relates to a method of preparing an aqueous topical composition according to the invention, wherein said at least one thickening agent is one or more of carbomers and wherein the dry composition comprises at least one buffer that is selected from one or more of monovalent cation buffers. Monovalent cations buffers are preferred when using carbomers, as monovalent cation buffers control the swelling of the carbomers well.

An embodiment of the invention relates to a method of preparing an aqueous topical composition according the invention comprising
a) providing a dry composition of viable bacteria of at least one probiotic bacterial strain in an amount of at least 5·10⁶ CFU/g and optionally at least one buffer,
b) providing at least one water miscible solvent comprising at least one thickening agent selected from one or more of poloxamers to provide a mixture having a viscosity of about 100 mPa·s when the temperature is no more than 10°C, such as no more than 10-15°C and a viscosity of about 1000 mPa·s when the temperature is above 25°C, such as above 20-25°C, and
c) adding said dry composition of step a) to said mixture of step b)
to prepare said aqueous topical composition having a viscosity of no more than 1000 mPa·s or no more than 500 mPa·s when the temperature is no more than 10°C, such as no more than 20°C, and a viscosity of more than 2000 mPa·s when the temperature is above 30°C, such as above 20°C, and wherein said aqueous topical composition comprises an amount in the range of 1·10⁶-10¹¹ CFU/g of said viable bacteria of at least one probiotic bacterial strain.

An alternative aspect of the present invention relates to a method of preparing an aqueous topical composition comprising
a) providing a dry composition of at least one moisture sensitive agent which is not viable bacteria of at least one probiotic bacterial strain,
b) providing at least one water miscible solvent, and
c) adding said dry composition of step a) to said water miscible solvent of step b)
to prepare said aqueous topical composition comprising said at least one moisture sensitive agent. In an embodiment, said at least one moisture sensitive agent which is not viable bacteria of at least one probiotic bacterial strain is active in the aqueous topical composition for up to 14 days at 25°C/60%RH or 30°C/75%RH or 5°C.

In an alternative embodiment, said viable bacteria of at least one probiotic bacterial strain are active or alive in the aqueous topical composition for up to 5 minutes at 25°C/60%RH or 30°C/75%RH or 5°C, such as for up to 20 minutes at 25°C/60%RH or 30°C/75%RH or 5°C, such as up to 1 hour at 25°C/60%RH or 30°C/75%RH or 5°C, such as up to 4 hours at 25°C/60%RH or 30°C/75%RH or 5°C, such as up to 12 hours at 25°C/60%RH or 30°C/75%RH or 5°C, such as up to 24 hours at 25°C/60%RH or 30°C/75%RH or 5°C, such as up to 2 days at 25°C/60%RH or 30°C/75%RH or 5°C, such as up to 3 days at 25°C/60%RH or 30°C/75%RH or 5°C, such as up to 4 days at 25°C/60%RH or 30°C/75%RH or 5°C, such as up to 5 days at 25°C/60%RH or 30°C/75%RH or 5°C, such as up to 6 days at 25°C/60%RH or 30°C/75%RH or 5°C, such as up to 7 days at 25°C/60%RH or 30°C/75%RH or 5°C, such as up to 8 days at 25°C/60%RH or 30°C/75%RH or 5°C, such as up to 9 days at 25°C/60%RH or 30°C/75%RH or 5°C, such as up to 10 days at 25°C/60%RH or 30°C/75%RH or 5°C, such as up to 14 days at 25°C/60%RH or 30°C/75%RH or 5°C, such as up to 18 days at 25°C/60%RH or 30°C/75%RH or 5°C, such as up to 22 days at 25°C/60%RH or 30°C/75%RH or 5°C, such as up to 26 days at 25°C/60%RH or 30°C/75%RH or 5°C, such as up to 30 days at 25°C/60%RH or 30°C/75%RH or 5°C, such as up to 2 months at 25°C/60%RH or 30°C/75%RH or 5°C.

Another aspect of the present invention relates to an aqueous topical composition comprising viable bacteria of at least one probiotic bacterial strain and at least one water miscible solvent, wherein an amount in the range of 1·10⁶-10¹¹ CFU/g of the viable bacteria of at least one probiotic bacterial strain is viable in the aqueous topical composition for a period of time, such as at least 5 minutes at 25°C/60%RH or 30°C/75%RH or 5°C, such as for at least 20 minutes at 25°C/60%RH or 30°C/75%RH or 5°C, such as at least 1 hour at 25°C/60%RH or 30°C/75%RH or 5°C, such as at least 4 hours at 25°C/60%RH or 30°C/75%RH or 5°C, such as at least 12 hours at 25°C/60%RH or 30°C/75%RH or 5°C, such as at least 24 hours at 25°C/60%RH or 30°C/75%RH or 5°C, such as at least 2 days at 25°C/60%RH or 30°C/75%RH or 5°C, such as at least 3 days at 25°C/60%RH or 30°C/75%RH or 5°C, such as at least 4 days at 25°C/60%RH or 30°C/75%RH or 5°C, such as at least 5 days at 25°C/60%RH or 30°C/75%RH or 5°C, such as at least 6 days at 25°C/60%RH or 30°C/75%RH or 5°C, such as at least 7 days at 25°C/60%RH or 30°C/75%RH or 5°C, such as at least 8 days at 25°C/60%RH or 30°C/75%RH or 5°C, such as at least 9 days at 25°C/60%RH or 30°C/75%RH or 5°C, such as at least 10 days at 25°C/60%RH or 30°C/75%RH or 5°C, such as at least 14 days at 25°C/60%RH or 30°C/75%RH or 5°C, such as for up to 5 minutes at 25°C/60%RH or 30°C/75%RH or 5°C, such as for up to 20 minutes at 25°C/60%RH or 30°C/75%RH or 5°C, such as up to 1 hour at 25°C/60%RH or 30°C/75%RH or 5°C, such as up to 4 hours at 25°C/60%RH or 30°C/75%RH or 5°C, such as up to 12 hours at 25°C/60%RH or 30°C/75%RH or 5°C, such as up to 24 hours at 25°C/60%RH or 30°C/75%RH or 5°C, such as up to 2 days at 25°C/60%RH or 30°C/75%RH or 5°C, such as up to 3 days at 25°C/60%RH or 30°C/75%RH or 5°C, such as up to 4 days at 25°C/60%RH or 30°C/75%RH or 5°C, such as up to 5 days at 25°C/60%RH or 30°C/75%RH or 5°C, such as up to 6 days at 25°C/60%RH or 30°C/75%RH or 5°C, such as up to 7 days at 25°C/60%RH or 30°C/75%RH or 5°C, such as up to 8 days at 25°C/60%RH or 30°C/75%RH or 5°C, such as up to 9 days at 25°C/60%RH or 30°C/75%RH or 5°C, such as up to 10 days at 25°C/60%RH or 30°C/75%RH or 5°C, such as up to 14 days at 25°C/60%RH or 30°C/75%RH or 5°C, such as up to 18 days at 25°C/60%RH or 30°C/75%RH or 5°C, such as up to 22 days at 25°C/60%RH or 30°C/75%RH or 5°C, such as up to 26 days at 25°C/60%RH or 30°C/75%RH or 5°C, such as up to 30 days at 25°C/60%RH or 30°C/75%RH or 5°C, such as up to 2 months at 25°C/60%RH or 30°C/75%RH or 5°C.

Another aspect of the present invention relates to an aqueous topical composition comprising viable bacteria of at least one probiotic bacterial strain and at least one water miscible solvent, wherein an amount in the range of 1·10⁶-10¹¹ CFU/g of the viable bacteria of at least one probiotic bacterial strain is viable in the aqueous topical composition for a period of time, such as 5 minutes to 2 months, such as 5 minutes to 1 month, such as 5 minutes to 14 days, such as 5 minutes to 7 days, such as 5 minutes to 4 days.

In an embodiment of the invention, at least 1·10⁶ CFU/g, such as at least 5·10⁶ CFU/g such as at least 10⁷ CFU/g, such as at least 10⁸ CFU/g, such as at least 10⁹ CFU/g, such as at least 10¹⁰, such as at least 10¹¹ CFU/g of said viable bacteria of at least one probiotic bacterial strain are viable in the aqueous topical composition for up to 14 days at 25°C/60%RH or 30°C/75%RH or 5°C.

It has surprisingly been shown that said probiotic bacteria remain viable in said aqueous topical composition for at least 5 minutes at 25°C/60%RH or 30°C/75%RH or 5°C. Additionally, and even more surprisingly, said probiotic bacteria remain viable in said aqueous topical composition for at least 7 or even 14 days at 25°C/60%RH or 30°C/75%RH or 5°C. This provides the consumer with the advantage of an aqueous topical composition being stable and having a high amount of viable bacteria of at least one probiotic bacterial strain.

An embodiment of the invention relates to an aqueous topical composition according to the invention, wherein said viable bacteria of at least one probiotic bacterial strain is provided in a dry composition comprising viable bacteria of at least one probiotic bacterial strain in an amount of at least 5·10⁶ CFU/g.

An embodiment of the invention relates to an aqueous topical composition according to the invention wherein said composition comprises one or more active components.

An embodiment of the invention relates to an aqueous topical composition according to the invention wherein said composition comprises at least one thickening agent and/or at least one buffer. When using said thickening agent and/or at least one buffer in said aqueous topical composition according to the invention, it has surprisingly been shown that said probiotic bacteria remain viable in said aqueous topical composition for at least 5 minutes at 25°C/60%RH or 30°C/75%RH or 5°C. Additionally, and even more surprisingly, said probiotic bacteria remain viable in said aqueous topical composition for at least 7 or even at least 14 days at 25°C/60%RH or 30°C/75%RH or 5°C or for up to 7 or even up to 14 days at 25°C/60%RH or 30°C/75%RH or 5°C. This provides the consumer with the advantage of an aqueous topical composition being stable and having a high amount of viable bacteria of at least one probiotic bacterial strain. Also, said aqueous topical composition has a very agreeable storage time/shelf life of at least 7 or even at least 14 days at 25°C/60%RH or 30°C/75%RH or 5°C or it has a very agreeable storage time for up to 7 or even up to 14 days at 25°C/60%RH or 30°C/75%RH or 5°C thereby providing a very useful product for the consumer.

An embodiment of the invention relates to an aqueous topical composition according to the invention, wherein said composition comprises at least one carbomer and at least one buffer and optionally at least one acid and/or at least one base.

An embodiment of the invention relates to an aqueous topical composition according to the invention, wherein said composition comprises at least one poloxamer.

An embodiment of the invention relates to an aqueous topical composition according to the invention, wherein said composition comprises at least one emulsifier.

An embodiment of the invention relates to an aqueous topical composition according to the invention, wherein said composition comprises at least one buffer.

An embodiment of the invention relates to an aqueous topical composition according to the invention, wherein said composition comprises one or more hydrophobic compounds.

An embodiment of the invention relates to an aqueous topical composition according to the invention, wherein said hydrophobic compound is selected from one or more of oils and waxes that are solid and/or fluid at room temperature.

An embodiment of the invention relates to an aqueous topical composition according to the invention, wherein said composition has a pH of 3.0-8.0 and a viscosity of more than 2000 mPa·s, such as a viscosity of more than 2250 mPa·s, such as a viscosity of more than 2500 mPa·s, such as a viscosity of more than 2700 mPa·s, such as more than 3000 mPa·s, such as more than 3300 mPa·s, such as more than 3600 mPa·s, such as more than 3900 mPa·s, such as more than 4000 mPa·s, such as more than 5000 mPa·s, such as more than 6000 mPa·s, such as more than 7500 mPa·s, such as more than 8500 mPa·s, such as more than 10000 mPa·s. In an embodiment of the invention, the pH may be 3.0-4.0, such as 3.5-4.5, such as 4.5-5.5, such as more than 3.0, such as more than 3.5, such as more than 4.0 such as more than 5.0, such as more than 5.5.

An embodiment of the invention relates to an aqueous topical composition according to the invention, wherein the viable bacteria of at least one probiotic bacterial strain is selected from one or more of a single strain or combination of strains of any one of the strains of *Lactococcus lactis* subsp. *lactis biovar. diacetylactis, Lactococcus lactis* subsp. *cremoris, Lactococcus lactis* subsp. *lactis,* any strain belonging to the genus Lactobacillus including but not limited to *Lactobacillus acidophilus, Lactobacillus casei* subsp. *casei, L. casei (Gynophilus), L. coleohominis, Lactobacillus delbrueckii* subsp. *bulgaricus, Lactobacillus crispatus, Lactobacillus fermentum, L. fornicalis, L. gallinarum, Lactobacillus gasseri, Lactobacillus helveticus, Lactobacillus iners, Lactobacillus jensenii, Lactobacillus lactis, L. mucosae, L. paracasei, L plantarum, L. salivarius, L. reuteri, Lactobacillus rhamnosus, Lactobacillus salivarius, L. vaginalis,* any strain belonging to the genus Bifidobacterium including but not limited to *Bifidobacterium adolescentis, Bifidobacterium angulatum, Bifidobacterium animalis* subsp. *lactis, Bifidobacterium bifidum, Bifidobacterium breve, Bifidobacterium catenulatum, Bifidobacterium dentium, Bifidobacterium infantis, Bifidobacterium longum, Bifidobacterium magnum, Bifidobacterium pseudocatenulatum,* or from the genera of Akkermansia, Anaerostipes, Butyricicoccus, Christensenella, Clostridia, Coprococcus, Dorea, Eubacterium, Faecalibacterium, Cutibacterium, such as *Cutibacterium acnes,* or Roseburia, Staphylococcus, such as *Staphylococcus epidermis* or *Staphylococcus hominis, Weissella viridescens,* or the family Coriobacteriaceae.

An embodiment of the invention relates to an aqueous topical composition according to the invention, wherein the viable bacteria of at least one probiotic bacterial strain is selected from one or more of a single strain or combination of strains belonging to the genus Bifidobacterium including but not limited to *Bifidobacterium animalis* subsp. *lactis,* such as *Bifidobacterium animalis* subsp. *lactis, Bifidobacterium breve,* and *Bifidobacterium infantis,* strains belonging to the genus Cutibacterium, such as *Cutibacterium acnes,* any strain belonging to the genus Lactobacillus including but not limited to *L. casei (Gynophilus), L. coleohominis, L. delbrueckii, L. fornicalis, L. gallinarum, L. iners, L. mucosae, L. paracasei,* such as *Lactobacillus paracasei* subsp. *paracasei, L plantarum, L. rhamnosus,* such as *Lactobacillus rhamnosus, L. salivarius, L. reuteri, L. vaginalis,* strains of the genus Staphylococcus, such as *Staphylococcus epidermis* and *Staphylococcus hominis,* and *Weissella viridescens.*

In a preferred embodiment, the probiotic strain to be included in the dry composition or aqueous topical composition according to the invention is selected from one or more of the strains belonging to the genus Bifidobacterium including but not limited to *Bifidobacterium animalis* subsp. *lactis,* such as *Bifidobacterium animalis* subsp. *lactis, Bifidobacterium breve,* and *Bifidobacterium infantis,* strains belonging to the genus Cutibaterium, such as *Cutibacterium acnes,* any strain belonging to the genus Lactobacillus including but not limited to *L. casei (Gynophilus), L. coleohominis, L. delbrueckii, L. fornicalis, L. gallinarum, L. iners, L. mucosae, L. paracasei,* such as *Lactobacillus paracasei* subsp. *paracasei, L plantarum, L. rhamnosus,* such as *Lactobacillus rhamnosus, L. salivarius, L. reuteri, L. vaginalis,* strains of the genus Staphylococcus, such as *Staphylococcus epidermis* and *Staphylococcus hominis,* and *Weissella viridescens.*

In presently preferred embodiments one, two, three, four, five, six, seven or eight different strains are present in a composition according to the invention.

An alternative aspect of the present invention relates to an aqueous topical composition comprising at least one moisture sensitive agent which is not viable bacteria of at least one probiotic bacterial strain and at least one water miscible solvent, wherein at least 10% of the at least moisture sensitive agent is active in the aqueous topical composition for at least 5 minutes or up to 14 days at 25°C/60%RH or 30°C/75%RH or 5°C.

Yet another aspect of the present invention is to provide a probiotic bacteria delivery device comprising
- a first housing element comprising viable bacteria of at least one probiotic bacterial strain and optionally a desiccant, and
- a second housing element comprising at least one water miscible solvent,
wherein the amount of viable bacteria of at least one probiotic bacterial strain in said first housing element is at least 5·10⁶ CFU/g for up to 3 years at 25°C/60%RH or 30°C/75%RH or 5°C, such as at least 10⁷ CFU/g, such as at least 10⁸ CFU/g, such as at least 10⁹ CFU/g for up to 3 years at 25°C/60%RH or 30°C/75%RH or 5°C, such as for up till 30 months at 25°C/60%RH or 30°C/75%RH or 5°C, such as for up till 24 months at 25°C/60%RH or 30°C/75%RH or 5°C, such as for up till 18 months at 25°C/60%RH or 30°C/75%RH or 5°C, such as for up till 12 months at 25°C/60%RH or 30°C/75%RH or 5°C, such as for up till 6 months at 25°C/60%RH or 30°C/75%RH or 5°C, or such as for up till 3 months at 25°C/60%RH or 30°C/75%RH or 5°C.

An alternative aspect of the present invention is to provide a delivery device comprising
- a first housing element comprising at least one moisture sensitive component which is not viable bacteria of at least one probiotic bacterial strain, and
- a second housing element comprising at least one water miscible solvent,
wherein at least 10% of the moisture sensitive component remain active for up to 3 years at 25°C/60%RH or 30°C/75%RH or 5°C.

An embodiment of the invention relates to a delivery device according to the invention, wherein said first and second housing elements are separate elements.

An embodiment of the invention relates to a delivery device according to the invention, wherein said first housing element and said second housing element is each separately sealed with a lid, and wherein said housing elements and said lids each provide storage of said viable bacteria of at least one probiotic bacterial strain and said water miscible solvent, without any leakage of said viable bacteria of at least one probiotic bacterial strain and/or leakage of said water miscible solvent during storage.

An embodiment of the invention relates to a delivery device according to the invention, wherein said first and second housing elements both have lids that are re-attachable.

An embodiment of the invention relates to a delivery device according to the invention, wherein said second housing element is an aluminium, plastic or glass tube with a re-attachable screw lid or pump lid.

In an embodiment, a device of the invention is provided, wherein said device is a glass vial or plastic vial with a dropper, pipette or pump lid. Said plastic vial may comprise a high moisture barrier foil such as an aluminium inner lining.

An embodiment of the invention relates to a delivery device according to the invention, wherein said first and/or second housing element is a sachet and/or an ampule.

A preferred embodiment of the invention relates to a delivery device according to the invention, wherein said first housing element is an ampule or a sachet and, wherein said second housing element is an aluminium or plastic tube with a re-attachable screw lid or pump lid. In an even more preferred embodiment of the invention, said first housing element is a re-attachable plastic lid that is screwed onto a second housing element made of glass, wherein said re-attachable plastic lid have a membrane between the housing elements that can be broken so that the contents of the housing elements can be mixed while the delivery device is closed/sealed from the outside environment.

An embodiment of the invention relates to a delivery device according to the invention, wherein said viable bacteria of at least one probiotic bacterial strain is provided in a dry composition.

An embodiment of the invention relates to a delivery device according to the invention, wherein said first housing element comprises one or more active components and/or a desiccant.

An embodiment of the invention relates to a delivery device according to the invention, wherein said first housing element comprises one or more hydrophobic components.

An embodiment of the invention relates to a delivery device according to the invention, wherein said first housing element comprises one or more buffers.

An embodiment of the invention relates to a delivery device according to the invention, wherein said second housing element comprises one or more active components.

An embodiment of the invention relates to a delivery device according to the invention, wherein said second housing element comprises one or more hydrophobic components.

An embodiment of the invention relates to a delivery device according to the invention, wherein said second housing element comprises one or more thickening agents.

An embodiment of the invention relates to a delivery device according to the invention, wherein said second housing element comprises one or more emulsifiers.

An embodiment of the invention relates to a delivery device according to the invention, wherein the viable bacteria of at least one probiotic bacterial strain is selected from one or more of a single strain or combination of strains of any one of the strains belonging to *Lactococcus lactis* subsp. *lactis biovar. diacetylactis, Lactococcus lactis* subsp. *cremoris, Lactococcus lactis* subsp. *lactis,* any strain belonging to the genus Lactobacillus including but not limited to *Lactobacillus acidophilus, Lactobacillus casei* subsp. *casei, L. casei (Gynophilus), L. coleohominis, Lactobacillus delbrueckii* subsp. *bulgaricus, Lactobacillus crispatus, Lactobacillus fermentum, L. fornicalis, L. gallinarum, Lactobacillus gasseri, Lactobacillus helveticus, Lactobacillus iners, Lactobacillus jensenii, Lactobacillus lactis, L. mucosae, L. paracasei, L plantarum, L. salivarius, L. reuteri, Lactobacillus rhamnosus, Lactobacillus salivarius, L. vaginalis,* any strain belonging to the genus Bifidobacterium including but not limited to *Bifidobacterium adolescentis, Bifidobacterium angulatum, Bifidobacterium animalis* subsp. *lactis, Bifidobacterium bifidum, Bifidobacterium breve, Bifidobacterium catenulatum, Bifidobacterium dentium, Bifidobacterium infantis, Bifidobacterium longum, Bifidobacterium magnum, Bifidobacterium pseudocatenulatum,* or from the genera of Akkermansia, Anaerostipes, Butyricicoccus, Christensenella, Clostridia, Coprococcus, Dorea, Eubacterium, Faecalibacterium, Cutibacterium, such as *Cutibacterium acnes,* or Roseburia, Staphylococcus, such as *Staphylococcus epidermis* or *Staphylococcus hominis, Weissella viridescens,* or the family Coriobacteriaceae.

An embodiment of the invention relates to a delivery device according to the invention, wherein the viable bacteria of at least one probiotic bacterial strain is selected from one or more of a single strain or combination of strains of any one of the genus Bifidobacterium including but not limited to *Bifidobacterium animalis* subsp. *lactis,* such as *Bifidobacterium animalis* subsp. *lactis, Bifidobacterium breve,* and *Bifidobacterium infantis,* strains belonging to the genus Cutibacterium, such as *Cutibacterium acnes,* any strain belonging to the genus Lactobacillus including but not limited to *L. casei (Gynophilus), L. coleohominis, L. delbrueckii, L. fornicalis, L. gallinarum, L. iners, L. mucosae, L. paracasei,* such as *Lactobacillus paracasei* subsp. *paracasei, L plantarum, L. rhamnosus,* such as *Lactobacillus rhamnosus, L. salivarius, L. reuteri, L. vaginalis,* strains of the genus Staphylococcus, such as *Staphylococcus epidermis* and *Staphylococcus hominis,* and *Weissella viridescens.*

In presently preferred embodiments one, two, three, four, five, six, seven or eight different strains are present in a composition according to the invention.

An embodiment of the invention relates to a delivery device according to the invention, wherein said first housing element maintains the water activity of said viable bacteria of at least one probiotic bacterial strain at a level of no more than 0.3 for up until 3 years at 25°C/60%RH or 30°C/75%RH or 5°C.

An embodiment of the invention relates to a delivery device according to the invention, wherein said first and second housing elements are connected in a male-female configuration.

An embodiment of the invention relates to a delivery device according to the invention, wherein said male-female configuration comprises a snugly fitted configuration.

Still another aspect of the present invention is to provide a method of preparing a delivery device according to the invention comprising the steps of
a) providing in a first housing element viable bacteria of at least one probiotic bacterial strain, and
b) providing in a second housing element at least one water miscible solvent to provide said delivery device.

An embodiment of the invention relates to a method of preparing a delivery device according to the invention, wherein said first housing element comprises one or more buffers.

An embodiment of the invention relates to a method of preparing a delivery device according to the invention, wherein said first housing element comprises one or more active components and/or a desiccant.

An embodiment of the invention relates to a method of preparing a delivery device according to the invention, wherein said first housing element comprises one or more hydrophobic components.

An embodiment of the invention relates to a method of preparing a delivery device according to the invention, wherein said second housing element comprises one or more active components.

An embodiment of the invention relates to a method of preparing a delivery device according to the invention, wherein said second housing element comprises one or more hydrophobic components.

An embodiment of the invention relates to a method of preparing a delivery device according to the invention, wherein said second housing element comprises one or more thickening agents and/or one or more emulsifiers.

An embodiment of the invention relates to a method of preparing a delivery device according to the invention, wherein the amount of viable bacteria of at least one probiotic bacterial strain in said delivery device is at least 5·10⁶ CFU/g for up to 3 years at 25°C/60%RH or 30°C/75%RH or 5°C, such as at least 10⁷ CFU/g, such as at least 10⁸ CFU/g, such as at least 10⁹ CFU/g for up to 3 years at 25°C/60%RH or 30°C/75%RH or 5°C, such as for up till 30 months at 25°C/60%RH or 30°C/75%RH or 5°C, such as for up till 24 months at 25°C/60%RH or 30°C/75%RH or 5°C, such as for up till 18 months at 25°C/60%RH or 30°C/75%RH or 5°C, such as for up till 12 months at 25°C/60%RH or 30°C/75%RH or 5°C, such as for up till 6 months at 25°C/60%RH or 30°C/75%RH or 5°C, or such as for up till 3 months at 25°C/60%RH or 30°C/75%RH or 5°C.

An embodiment of the invention relates to a method of preparing a delivery device according to the invention, wherein the viable bacteria of at least one probiotic bacterial strain is selected from one or more of a single strain or combination of strains of any one of the strains belonging to *Lactococcus lactis* subsp. *lactis biovar. diacetylactis, Lactococcus lactis* subsp. *cremoris, Lactococcus lactis* subsp. *lactis,* any strain belonging to the genus Lactobacillus including but not limited to *Lactobacillus acidophilus, Lactobacillus casei* subsp. *casei, L. casei (Gynophilus), L. coleohominis, Lactobacillus delbrueckii* subsp. *bulgaricus, Lactobacillus crispatus, Lactobacillus fermentum, L. fornicalis, L. gallinarum, Lactobacillus gasseri, Lactobacillus helveticus, Lactobacillus iners, Lactobacillus jensenii, Lactobacillus lactis, L. mucosae, L. paracasei, L plantarum, L. salivarius, L. reuteri, Lactobacillus rhamnosus, Lactobacillus salivarius, L. vaginalis,* any strain belonging to the genus Bifidobacterium including but not limited to *Bifidobacterium adolescentis, Bifidobacterium angulatum, Bifidobacterium animalis* subsp. *lactis, Bifidobacterium bifidum, Bifidobacterium breve, Bifidobacterium catenulatum, Bifidobacterium dentium, Bifidobacterium infantis, Bifidobacterium longum, Bifidobacterium magnum, Bifidobacterium pseudocatenulatum,* or from the genera of Akkermansia, Anaerostipes, Butyricicoccus, Christensenella, Clostridia, Coprococcus, Dorea, Eubacterium, Faecalibacterium, Cutibacterium, such as *Cutibacterium acnes,* or Roseburia, Staphylococcus, such as *Staphylococcus epidermis* or *Staphylococcus hominis, Weissella viridescens,* or the family Coriobacteriaceae.

An embodiment of the invention relates to a method of preparing a delivery device according to the invention, wherein the viable bacteria of at least one probiotic bacterial strain is selected from one or more of a single strain or combination of strains of any one of the genus Bifidobacterium including but not limited to *Bifidobacterium animalis* subsp. *lactis,* such as *Bifidobacterium animalis* subsp. *lactis, Bifidobacterium breve,* and *Bifidobacterium infantis,* strains belonging to the genus Cutibacterium, such as *Cutibacterium acnes,* any strain belonging to the genus Lactobacillus including but not limited to *L. casei (Gynophilus), L. coleohominis, L. delbrueckii, L. fornicalis, L. gallinarum, L. iners, L. mucosae, L. paracasei,* such as *Lactobacillus paracasei* subsp. *paracasei, L plantarum, L. rhamnosus,* such as *Lactobacillus rhamnosus, L. salivarius, L. reuteri, L. vaginalis,* strains of the genus Staphylococcus, such as *Staphylococcus epidermis* and *Staphylococcus hominis,* and *Weissella viridescens.*

In presently preferred embodiments one, two, three, four, five, six, seven or eight different strains are present in a composition according to the invention.

An embodiment of the invention relates to a method of preparing a delivery device according to the invention, wherein said first housing element maintains the water activity of said viable bacteria of at least one probiotic bacterial strain at a level of no more than 0.3 for up to 3 years at 25°C/60%RH or 30°C/75%RH or 5°C, such as at least 10⁷ CFU/g, such as at least 10⁸ CFU/g, such as at least 10⁹ CFU/g for up to 3 years at 25°C/60%RH or 30°C/75%RH or 5°C, such as for up till 30 months at 25°C/60%RH or 30°C/75%RH or 5°C, such as for up till 24 months at 25°C/60%RH or 30°C/75%RH or 5°C, such as for up till 18 months at 25°C/60%RH or 30°C/75%RH or 5°C, such as for up till 12 months at 25°C/60%RH or 30°C/75%RH or 5°C, such as for up till 6 months at 25°C/60%RH or 30°C/75%RH or 5°C, or such as for up till 3 months at 25°C/60%RH or 30°C/75%RH or 5°C.

An even further aspect of the present invention relates to the use of a delivery device according to the invention for providing viable bacteria of at least one probiotic bacterial strain in an aqueous topical composition according to the invention for at least 14 days at 25°C/60%RH or 30°C/75%RH or 5°C, such as for at least 5 minutes at 25°C/60%RH or 30°C/75%RH or 5°C, such as for at least 20 minutes at 25°C/60%RH or 30°C/75%RH or 5°C, such as at least 1 hour at 25°C/60%RH or 30°C/75%RH or 5°C, such as at least 4 hours at 25°C/60%RH or 30°C/75%RH or 5°C, such as at least 12 hours at 25°C/60%RH or 30°C/75%RH or 5°C, such as at least 24 hours at 25°C/60%RH or 30°C/75%RH or 5°C, such as at least 2 days at 25°C/60%RH or 30°C/75%RH or 5°C, such as at least 3 days at 25°C/60%RH or 30°C/75%RH or 5°C, such as at least 4 days at 25°C/60%RH or 30°C/75%RH or 5°C, such as at least 5 days at 25°C/60%RH or 30°C/75%RH or 5°C, such as at least 6 days at 25°C/60%RH or 30°C/75%RH or 5°C, such as at least 7 days at 25°C/60%RH or 30°C/75%RH or 5°C, such as at least 8 days at 25°C/60%RH or 30°C/75%RH or 5°C, such as at least 9 days at 25°C/60%RH or 30°C/75%RH or 5°C, such as at least 10 days at 25°C/60%RH or 30°C/75%RH or 5°C, such as at least 14 days at 25°C/60%RH or 30°C/75%RH or 5°C, such as for up to 5 minutes at 25°C/60%RH or 30°C/75%RH or 5°C, such as for up to 20 minutes at 25°C/60%RH or 30°C/75%RH or 5°C, such as up to 1 hour at 25°C/60%RH or 30°C/75%RH or 5°C, such as up to 4 hours at 25°C/60%RH or 30°C/75%RH or 5°C, such as up to 12 hours at 25°C/60%RH or 30°C/75%RH or 5°C, such as up to 24 hours at 25°C/60%RH or 30°C/75%RH or 5°C, such as up to 2 days at 25°C/60%RH or 30°C/75%RH or 5°C, such as up to 3 days at 25°C/60%RH or 30°C/75%RH or 5°C, such as up to 4 days at 25°C/60%RH or 30°C/75%RH or 5°C, such as up to 5 days at 25°C/60%RH or 30°C/75%RH or 5°C, such as up to 6 days at 25°C/60%RH or 30°C/75%RH or 5°C, such as up to 7 days at 25°C/60%RH or 30°C/75%RH or 5°C, such as up to 8 days at 25°C/60%RH or 30°C/75%RH or 5°C, such as up to 9 days at 25°C/60%RH or 30°C/75%RH or 5°C, such as up to 10 days at 25°C/60%RH or 30°C/75%RH or 5°C, such as for up to 14 days at 25°C/60%RH or 30°C/75%RH or 5°C, such as up to 18 days at 25°C/60%RH or 30°C/75%RH or 5°C, such as up to 22 days at 25°C/60%RH or 30°C/75%RH or 5°C, such as up to 26 days at 25°C/60%RH or 30°C/75%RH or 5°C, such as up to 30 days at 25°C/60%RH or 30°C/75%RH or 5°C, such as up to 2 months at 25°C/60%RH or 30°C/75%RH or 5°C.

When the consumer requires to be able to obtain the finished aqueous topical composition of the invention, the contents of the housing elements are simply combined i.e. by mixing, shaking and/or stirring. Thus, when the consumer uses the delivery device according to the invention, the consumer mixes the contents of the delivery device and thus provides an aqueous topical composition according to the invention having at least 1·10⁶ viable bacteria of at least one probiotic bacterial strain for up to 14 days at 25°C/60%RH or 30°C/75%RH or 5°C. In another embodiment the consumer mixes the contents of the delivery device and thus provides an aqueous topical composition according to the invention having at least 1·10⁶ viable bacteria of at least one probiotic bacterial strain for at least 14 days at 25°C/60%RH or 30°C/75%RH or 5°C.

An embodiment of the present invention relates to the use of a delivery device according to the invention for providing at least at least 1·10⁶ of one viable bacteria of at least one probiotic bacterial strain in an aqueous topical composition according to the invention for up to 14 days at 25°C/60%RH or 30°C/75%RH or 5°C.

### Definitions

Prior to discussing the present invention in further details, the following terms and conventions will first be defined:

### Probiotic bacteria or probiotic bacterial strain

In the present context, "probiotic bacteria" or "bacteria of a probiotic bacterial strain" refer to a culture of microorganisms, such as bacteria, which, when applied to man or animal, beneficially affects the host (FAO/WHO, 2001, Health and Nutritional Properties of Probiotics in Food including Powder Milk with Live Lactic Acid Bacteria. Report of a Joint FAO/WHO Expert Consultation on Evaluation of Health and Nutritional Properties of Probiotics in Food Including Powder Milk with Live Lactic Acid Bacteria). Unless the context indicates otherwise, the terms "microorganism", "bacterium" and "probiotic" are used interchangeably. A bacterial "strain" as used herein refers to a bacterium which remains genetically unchanged when grown or multiplied.

In a preferred embodiment, the probiotic strain to be included in the dry composition or aqueous topical composition according to the invention is selected from one or more of a single strain or combination of strains of any one of the genus Bifidobacterium including but not limited to *Bifidobacterium animalis* subsp. *lactis,* such as *Bifidobacterium animalis* subsp. *lactis, Bifidobacterium breve,* and *Bifidobacterium infantis,* strains belonging to the genus Cutibacterium, such as *Cutibacterium acnes,* any strain belonging to the genus Lactobacillus including but not limited to *L. casei (Gynophilus), L. coleohominis, L. delbrueckii, L. fornicalis, L. gallinarum, L. iners, L. mucosae, L. paracasei,* such as *Lactobacillus paracasei* subsp. *paracasei, L plantarum, L. rhamnosus,* such as *Lactobacillus rhamnosus, L. salivarius, L. reuteri, L. vaginalis,* strains of the genus Staphylococcus, such as *Staphylococcus epidermis* and *Staphylococcus hominis,* and *Weissella viridescens.*

### Topical and aqueous topical composition

In the present context, "topical" is intended to mean application to a particular surface on or in the body, such as application to an external or internal body surface such as the skin or a mucous membrane, such as an ear, inside the nose, inside the mouth, lip, the urogenital area such as the urethral, vaginal, or rectal area, including the urethral opening, the vaginal opening and the anus. In a preferred embodiment, said topical application is intended for the urogenital area such as the urethral, vaginal, or rectal area, including the urethral opening, the vaginal opening and the anus. In another preferred embodiment, said topical application is intended for the skin.

In an embodiment of the invention, said skin or mucous membrane may be intact skin or mucous membrane. In another embodiment, said skin or mucous membrane may be compromised or damaged skin, e.g. suffering from dermatitis, such as atopic dermatitis, or having a burn, eczema, one or more psoriatic plaques, wounds, acne, sensitive skin, itching skin, vitiligo, rosacea, lichen sclerosus.

Thus, in the present context, "aqueous topical composition" is intended to mean a topical composition comprising at least one water miscible solvent and viable bacteria of at least one probiotic bacterial strain that are capable of surviving in said aqueous topical composition until topical application, i.e. for at least 5 minutes at 25°C/60%RH or 30°C/75%RH or 5°C, such as for at least 20 minutes at 25°C/60%RH or 30°C/75%RH or 5°C, such as at least 1 hour at 25°C/60%RH or 30°C/75%RH or 5°C, such as at least 4 hours at 25°C/60%RH or 30°C/75%RH or 5°C, such as at least 12 hours at 25°C/60%RH or 30°C/75%RH or 5°C, such as at least 24 hours at 25°C/60%RH or 30°C/75%RH or 5°C, such as at least 2 days at 25°C/60%RH or 30°C/75%RH or 5°C, such as at least 3 days at 25°C/60%RH or 30°C/75%RH or 5°C, such as at least 4 days at 25°C/60%RH or 30°C/75%RH or 5°C, such as at least 5 days at 25°C/60%RH or 30°C/75%RH or 5°C, such as at least 6 days at 25°C/60%RH or 30°C/75%RH or 5°C, such as at least 7 days at 25°C/60%RH or 30°C/75%RH or 5°C, such as at least 8 days at 25°C/60%RH or 30°C/75%RH or 5°C, such as at least 9 days at 25°C/60%RH or 30°C/75%RH or 5°C, such as at least 10 days at 25°C/60%RH or 30°C/75%RH or 5°C, such as at least 14 days at 25°C/60%RH or 30°C/75%RH or 5°C or additionally, for up to 5 minutes at 25°C/60%RH or 30°C/75%RH or 5°C, such as for up to 20 minutes at 25°C/60%RH or 30°C/75%RH or 5°C, such as up to 1 hour at 25°C/60%RH or 30°C/75%RH or 5°C, such as up to 4 hours at 25°C/60%RH or 30°C/75%RH or 5°C, such as up to 12 hours at 25°C/60%RH or 30°C/75%RH or 5°C, such as up to 24 hours at 25°C/60%RH or 30°C/75%RH or 5°C, such as up to 2 days at 25°C/60%RH or 30°C/75%RH or 5°C, such as up to 3 days at 25°C/60%RH or 30°C/75%RH or 5°C, such as up to 4 days at 25°C/60%RH or 30°C/75%RH or 5°C, such as up to 5 days at 25°C/60%RH or 30°C/75%RH or 5°C, such as up to 6 days at 25°C/60%RH or 30°C/75%RH or 5°C, such as up to 7 days at 25°C/60%RH or 30°C/75%RH or 5°C, such as up to 8 days at 25°C/60%RH or 30°C/75%RH or 5°C, such as up to 9 days at 25°C/60%RH or 30°C/75%RH or 5°C, such as up to 10 days at 25°C/60%RH or 30°C/75%RH or 5°C, such as up to 14 days at 25°C/60%RH or 30°C/75%RH or 5°C, such as up to 18 days at 25°C/60%RH or 30°C/75%RH or 5°C, such as up to 22 days at 25°C/60%RH or 30°C/75%RH or 5°C, such as up to 26 days at 25°C/60%RH or 30°C/75%RH or 5°C, such as up to 30 days at 25°C/60%RH or 30°C/75%RH or 5°C, such as up to 2 months at 25°C/60%RH or 30°C/75%RH or 5°C, or, alternatively for 5 minutes to 2 months, such as 5 minute to 1 month, such as 5 minute to 14 days, such as 5 minutes to 7 days, such as 5 minutes to 4 days.

Said aqueous topical composition is suitable for topical use in a mammal, particularly a human. All components in the aqueous topical composition of the invention must be suitable for application to the body surface in question, i.e. they must be approved for topical application to the body surface in the concentrations used and/or be dermatologically and cosmetologically acceptable.

An aqueous topical composition according to the invention may be administered to a mammal, preferably a human, via a large range of topical administration forms. In the present context, topical compositions are epicutaneous, meaning that they are applied directly to the skin or mucous membranes.

An aqueous topical composition of the invention may be formulated as a cream, drop, foam, gel, lotion, mousse, ointment, paste, poultice, powder, salve, serum, spray, thickened formulation, toothpaste or unguent. In a preferred embodiment, the aqueous topical composition of the invention is selected from a cream, gel, or a lotion.

### Dry composition

In the present context, a "dry composition" is intended to mean that the probiotic bacteria of the present invention initially is provided in a dry composition that is powdered having a water activity of no more than 0.30 and a particle size distribution below 500µm. In an embodiment, an aqueous topical composition of the invention is provided, wherein said dry composition has a diameter of no more than 500 µm, such as no more than 400 µm, such as no more than 300 µm, such as no more than 200 µm, such as no more than 150 µm, such as no more than 100 µm. In a preferred embodiment, said dry composition has a diameter of no more than 200 µm. Said dry composition has been prepared by conventional methods of freeze drying and/or spray drying followed by conventional grinding and sieving to obtain the desired particle size distribution below 500µm.

A dry composition of the present invention comprises the bacteria in dried form, which can be obtained e.g. by freeze-drying, spray-drying or lyophilization.

Before the bacteria are dried, e.g. freeze-dried, they are generally mixed with a cryoprotectant in order to obtain a high viability. The term "a cryoprotectant" is used in the context of the present invention to refer to a substance that is able to improve the survival during freezing and/or drying and to improve the storage stability of bacteria. The cryoprotectant used herein preferably comprises a saccharide.

The saccharide may be a mono-, di-, oligo- or polysaccharide, or a mixture of at least two saccharides. Useful monosaccharides include glucose (also known as dextrose), fructose, ribose and galactose and useful disaccharides include among other sucrose, trehalose, maltose and lactose. The composition may comprise one or more mono- or disaccharides, such as one, two, or three or even more different saccharides.

As an example, the cryoprotectant may comprise a mixture of a disaccharide, such as sucrose, and a polysaccharide, such as maltodextrin.

The cryoprotectant may further comprise a peptide, protein, protein hydrolysate or a mixture thereof. Examples of peptides and proteins to be used are casein, pea, whey, albumin, soy protein, glutamic acid or gelatin, and any isolate or hydrolysate thereof. Other additives, e.g. antioxidants such as ascorbate, sodium citrate, propyl gallate may also be present.

A dry composition of the present invention can be defined as having "no available water" and "substantially free from water", and these words are used interchangeably and are intended to mean that the water present in the first housing element comprising viable bacteria of at least one probiotic bacterial strain is not able to solvate the probiotic bacteria, because the water is present in too small an amount. This also means that the water activity is no more than 0.30, such as no more than 0.20, such as no more than 0.15 in the dry composition. Preferably, the water activity is no more than 0.30 in the dry composition of the present invention.

In the present context, "anhydrous" viable bacteria of at least one probiotic bacterial strain means viable probiotic bacteria which are essentially free of water. Particularly, for such a viable bacteria of at least one probiotic bacterial strain a significant amount of water has not been added, and the amount of un-bound water in the viable bacteria of at least one probiotic bacterial strain is very low. Some components in the probiotic bacteria may include some bound water, i.e. water of crystallisation in the form of hydrates or sugars that have water bound to them. This water is however not immediately available for participating in hydrolysis reactions with water sensitive components such as freeze dried viable bacteria of at least one probiotic bacterial strain. In preferred embodiments the anhydrous viable bacteria of at least one probiotic bacterial strain of the present invention comprises less than 20%(w/w) water, such as less than 16%(w/w) water, such as less than 12%(w/w) water, such as less than 8%(w/w) water, such as less than 7%(w/w) water, such as less than 6%(w/w) water, such as less than 5%(w/w) water, such as less than 4%(w/w) water, 3%(w/w), 2%(w/w), 1.5%(w/w), 1%(w/w), 0.5%(w/w), such as less than 0.2% (w/w) water as compared to total composition weight. Preferably the amount of added water in an anhydrous viable bacteria of at least one probiotic bacterial strain is less than 3%(w/w), such as less 2%(w/w), 1%(w/w), 0.5%(w/w), such as less than 0.1%(w/w).

### Water activity

In the present context, "water activity" or "a_{w}" is the partial vapor pressure of water in a substance divided by the standard state partial vapor pressure of water. Pure distilled water has a water activity of exactly one. As temperature increases, a_{w} typically increases.

### Viable/viable bacteria of at least one probiotic bacterial strain

In the present context, "viable" is intended to mean that the probiotic bacteria of the present invention are capable of normal growth and development, i.e. viable bacteria of at least one probiotic bacterial strain means that the probiotic bacterial are viable and thus capable of normal growth and development. In the present invention, the viable bacteria of at least one probiotic bacterial strain have been dried in a way so as to leave the probiotic bacteria viable. Several ways of preparing viable bacteria of at least one probiotic bacterial strain are well known in the art.

### Buffers

In the present context, a "buffer" is an acid or base used to maintain the acidity (pH) of a solution near a chosen value after the addition of another acid or base. Hence, the buffers balance the pH of an aqueous topical composition of the invention to make the composition less irritating. The one or more buffers is/are selected from buffers with a pKa in the range of 2-9, such as 2-7. Presently preferred buffers are monovalent cations. In an even more preferred embodiment of the invention, the one or more buffers is/are selected from monovalent cations of carbonates, ascorbates, phosphates, malates, citrates, tartrates, such as sodium bicarbonate, sodium malate, sodium tartrate, potassium tartrate, sodium carbonate, and tri sodium citrate dehydrate. However, other ways of adjusting the pH in the aqueous topical composition may also be feasible. As an example, triethanolamine and sodium hydroxide may be useful for certain embodiments.

In an embodiment, one or more buffers is/are present in the dry composition and/or added to the water miscible solvent when desired to control the pH at a certain level. This may be important when controlling the viscosity when using e.g. certain carbomers according to the invention. In a preferred embodiment of the invention, one or more buffers are present in the final product i.e. the aqueous topical composition of the invention.

A buffer is important for the function of the carbomer in the present invention, as a carbomer has a low viscosity when the pH is low, as carbomers then do not swell (absorb and retain water). When the pH increases, the carbomer swells and thus increases the viscosity. Hence, a buffer in the presence of a carbomer provides for a possibility of controlling the viscosity.

### Water miscible solvent

In the present context, "water miscible solvent" is a solvent which may be mixed with water without any phase separation. Water miscible solvents particularly include hydrophilic solvents. Such solvents include for example lower alkyl alcohols, hydrophilic organic solvents and water. A hydrophile is a molecule or other molecular entity that is attracted to water molecules and tends to be dissolved by water. In the present context, "alkyl alcohol" is in the broadest sense an organic alcohol consisting of a branched or linear alkyl chain and one or more hydroxyl groups attached thereto. Thus, in a preferred embodiment the water miscible solvent is selected from the group consisting of a C2-C5 alkyl alcohol, glycerol, polyethylene glycol, propylene glycol and C2-C5 glycol. In a particularly preferred embodiment, the water miscible solvent is one or more of water, glycerol, polyethylene glycol and/or propylene glycol. In an even more preferred embodiment said water miscible solvent is water. The amount of said water is at least 60%(w/w), such as least 65%(w/w), such as least 70%(w/w), such as least 75%(w/w), such as least 80%(w/w), such as least 85%(w/w), such as least 90%(w/w), such as least 95%(w/w) in said mixture of the invention or in said aqueous topical composition of the invention. In the examples of the present invention, said water miscible solvent is water in an amount of more than 90%(w/w) of the aqueous topical composition of the invention. In a preferred embodiment, an acid or base can be added to the water miscible solvent to adjust the pH.

### Thickening agent

A "thickening agent" according to the invention is defined as a compound that is capable of increasing the viscosity of the aqueous topical composition as defined herein. A commercial homogeneous aqueous topical composition as defined herein for topical application is prepared with a suitable viscosity for application just before use. Suitable thickening agents are selected from one or more of compounds of polyacrylic acid (carbomer) or derivatives hereof or other fast gelling polymers e.g. hyaluronic acid, gelatine, pectin, carrageenan and methylcellulose. Preferred thickening agents are selected from one or more of polymers, carbomer, poloxamer, PEG/macrogol and/or carboxylic acid. In an even more preferred embodiment, thickening agents are selected from one or more types of acrylic acid and C10-30 alkyl acrylate co-monomer and crosslinked polyacrylic acid copolymer.

Carbomer solutions possess an important characteristic i.e. they have the ability to absorb and retain water and swell to many times their original volume. The degree of swelling is dependent on solution pH, so that the gelling occurs when the pH increases above a certain point, dependent on the particular carbomer used. Thus, if the aqueous topical composition of the present invention comprises carbomer and a pH where it swells, said aqueous topical composition will have a viscosity that makes it suitable for topical application.

Poloxamer solutions have an important characteristic i.e. they demonstrate temperature dependent self-assembling and thermo-gelling behaviour. This means that aqueous solutions of poloxamers are liquid at low temperature and form a gel at higher temperature in a reversible process. Thus, if the aqueous topical composition of the present invention comprises a poloxamer, said aqueous topical composition will have a low viscosity when the temperature is low, e.g. if keeping the aqueous topical composition in the refrigerator. Likewise, if the said aqueous topical composition is kept at room temperature, said aqueous topical composition will have a viscosity that makes it suitable for topical application.

In a preferred embodiment, the one or more thickening agent is present in the water miscible solvent, optionally with adjusted pH. In an even more preferred embodiment, the one or more thickening agent is one or more of a carbomer present in the water miscible solvent. In an alternative embodiment, the one or more thickening agent is one or more of a poloxamer present in the water miscible solvent.

### Mixture

According to the invention, "a mixture" is defined as the mixture that is obtained when at least one water miscible solvent is mixed with at least one thickening agent and optionally a buffer or other components as defined herein to provide a mixture that is thickened i.e. having a viscosity that is larger than the at least one water miscible solvent on its own.

### Viscosity

The dynamic viscosity of the water miscible solvent should be low to medium and fluid, i.e. preferably having a viscosity less than 2500 cP (mPa·s.) when measured at 20 rpm at 20°C/60%RH, such as less than 2400 cP (mPa·s.), such as less than 2200 cP (mPa·s.), such as less than 2000 cP (mPa·s.), such as less than 1000 cP (mPa·s.). When the water miscible solvent is mixed with the dry composition, the viscosity of the mixture increases 3-1000 times and becomes medium to high, i.e. 2500 cP (mPa·s.) or more when measured at 20 rpm at 20°C/60%RH, more preferably above 5000 cP (mPa·s.) when measured at 20 rpm 20°C/60%RH. The viscosity is measured using a conventional Brookfield Laboratory Viscometer. An even more precise measurement can be made using a HR-1 Discovery Hybrid Rheometer from TA Instruments, with a 40 mm parallel plate on Peltier plate steel, with a constant shear rate at 2.0 s⁻¹.

### CFU

In the present context, "CFU" is intended to mean "colony forming units" and Colonic Forming Units per g product, (CFU/g), can be measured as disclosed under the Examples section. Most gels, lotions and creams have a density of 0.9 g/ml to 1.1 g/ml, hence CFU/g is for all practical purposes identical with CFU/ml and values for the one are comparable to the other for primarily water-based compositions.

### Oils and waxes

In the present context, "oils and waxes", being a sub-group of hydrophobic compounds as used herein, are vegetal and/or mineral oils and/or waxes. In an embodiment, oils and waxes to be used in the composition of the invention are selected from oils and waxes that are solid or fluid at room temperature.

Oils and waxes that are fluid at room temperature may be selected from one or more of ethyl oleate, evening primrose oil, grapeseed oil, hydrogenated vegetable (palm) oil, isopropyl isostearate, isopropyl myristate, isopropyl palmitate, jojoba (seed) oil, liquid lanolin, macademia oil, medium chain triglycerides, olive oil, paraffin oil/mineral oil, pomegrante oil, rapseed oil, rice bran oil, rosehip oil, safflower oil, sesame oil, shea nut butter, soybean oil, sunflower oil, sweet almond oil, trimyristin, tripalmitin, tristearin, and avocado oil. In a preferred embodiment, the oil or wax that is fluid at room temperature is selected from one or more of paraffin oil, almond oil and jojoba oil. In an even more preferred embodiment, the oil and waxes that are fluid at room temperature is paraffin oil.

Oils and waxes that are solid at room temperature as used herein are selected from one or more of anionic emulsifying wax, candelilla wax, carnauba wax, cetyl palmitate, cocoa butter, gum arabic, hard fat, microcrystalline wax, nonionic emulsifying wax, paraffin, shea butter, synthetic beeswax, white wax, xantan gum, sunflower wax.

In a preferred embodiment, the oil or wax that is solid at room temperature is selected from one or more of anionic emulsifying wax, candelilla wax, carnauba wax, microcrystalline wax, non-ionic emulsifying wax, synthetic beeswax, white wax and/or sunflower wax. In an even more preferred embodiment, the oil and waxes that are solid at room temperature is selected from one or more of, cetyl palmitate, cocoa butter, gum Arabic, hard fat, paraffin, shea butter and/or xantan gum.

In an even more preferred embodiment, a combination of fluid and solid oils and waxes are used, such as at least one solid oil combined with at least one fluid oil, such as at least two fluid oils and at least one solid oil or vice versa, such as at least three fluid oils and at least one solid or vice versa.

### Delivery device

In the present context, the "delivery device" is intended to mean a device suitable for delivering of viable bacteria of at least one probiotic bacterial strain to a user of the device. More specifically, the delivering device comprises a first and a second housing element that provides an interface between the delivery device and the user.

According to the invention, the at least one probiotic bacteria is intended to be protected from contamination and/or moisture in a sealed housing element thereby providing a stable environment for long term storage of the probiotics (named the first housing element). Likewise, the at least one water miscible solvent is intended to be protected from outside contamination in a separate, sealed housing element thereby providing a stable environment for long term storage of the water miscible solvent (named second housing element). Hence, once the user is ready to use the aqueous topical composition of the invention, the seals on both housing elements are broken, and the contents of the housing elements are mixed. In an embodiment of the invention, the mixing may be performed in the first housing element or in the second housing element. The mixing may also be performed in a third housing element suitable for such mixing, such as a cup, glass, and spoon or in a hand.

If the mixing is performed in the first housing element, then, the first housing element also serves as a delivery device to a user of the device.

If the mixing is performed in the second housing element, then the second housing element also serves as a delivery device to a user of the device.

If the mixing is performed in a third housing element suitable for receiving and mixing the contents of the first and second housing elements, then the third housing element serves as a delivery device to a user of the device.

In a preferred embodiment, the first and the second housing elements are attached to each other during storage, mixing and delivery to a user of the device. In an alternative embodiment, the first, second and a third housing element are attached to each other during storage, mixing and delivery to a user of the device.

In another preferred embodiment, the first and the second housing elements are separate entities and the mixing is performed in a third suitable housing element, such as a cup, glass, and spoon or in a hand.

In an alternative embodiment, a delivering element, such as a pump may be located onto the first or the second or a third housing element and adapted for receiving the mixture of the viable bacteria of at least one probiotic bacterial strain from the first housing element and the least one water miscible solvent from the second housing element.

In some embodiments, a delivering element is configured in a way so it can be pushed onto the first or second or third housing element (push fit).

In some embodiments, a delivering element is configured in a way so it can be hinged onto the first or second or third housing element. In some embodiments, the delivering element is configured in a way so it can be screwed onto the first or second or third housing element. In a preferred embodiment, the delivering element is either the first or the second housing element.

A delivery device or housing element of the present invention may further comprise at least one opening for release of contents to the surroundings. Such openings may fit onto the top of a housing element of the invention and a lid may protect the contents of the housing elements from contaminants from the environment and prevent leakage of volatile substances to the surroundings.

### Housing element

In the present context, the outer casing of the delivery device is herein referred to as a "housing element". In an embodiment, no more than one housing element exist which entirely covers a second housing element, however preferably 2 housing elements exist separately, such as 3, such as 4. Preferably, a first and a second housing element exist as described herein either separately, or joined snugly either by a delivering element or joined to one another by means described herein, such as e.g. a re-attachable lid. The housing element is the component that the user holds to use the delivery device. The housing element may also serve as the delivery device on its own. The housing element provides protection of the contents inside the housing element from being in contact with the surroundings i.e. the housing element may protect the content of the housing element from contaminants from the environment and prevent leakage of substances to the surroundings. In an embodiment of the invention, the housing element comprises a desiccant.

In embodiments of the invention, the housing element is made from suitable materials, such as rubber, plastic (PE, PET), glass or metal, such as aluminium or steel. The materials may also have been coated e.g. a metal with a heat resistant coating, such as a rubber or plastic coating. The housing material may also be a sachet, an ampule or the like.

The housing element may have a height of less than 20 cm, such as less than 15 cm, preferably less than 12 cm, such as less than 10 cm, such as less than 8 cm, such as less than 6 cm, such as less than 4 cm and a diameter of less than 6 cm, such as less than 5 cm, such as less than 4 cm, such as less than 2 cm.

### RH

In the present context, "RH" is intended to mean "Relative humidity" and it is the ratio of the partial pressure of water vapor to the equilibrium vapor pressure of water at a given temperature.

### Active components

In the present context, an "active component" is defined as one or more of a compound selected from the group of vitamins, minerals, antiseptics, preservatives, sun protection agents, moisture sensitive agents or various other agents. Vitamins may be selected from vitamin A, such as retinol/retinyl esters, vitamin B such as nicotinamide/niacin, vitamin D such as vitamin D3 and/or vitamin E such as different types of tocopherol e.g. d-o-Tocopherol, dl-o-Tocopherol, γ-tocopherol or tocotrienols. Minerals may be selected from zinc and flour.

Antibacterials/antiseptics may be selected from salicylic acid, azelaic acid benzoyl peroxide. Antiseptics to be used for the present invention should not kill or inhibit growth of the probiotic bacteria of the present invention when the probiotic bacteria are in a dry state i.e. having a water activity of no more than 0.3. Also, in the aqueous topical composition of the present invention, the antiseptics should not kill or inhibit growth of the probiotic bacteria.

Preservatives may be selected from preservatives well known in the art, such as e.g. sorbic acid, sodium sorbate, benzoic acid, sodium benzoate and benzoates hydroxybenzoate, lactic acid, propionic acid, sodium propionate and parabens, such as one or more of methylparaben, ethylparaben, propylparaben, butylparaben, heptylparaben, isobutylparaben, isopropylparaben, benzylparaben and their sodium salts. In an embodiment of the invention, the preferred preservative is selected from one or more of sorbic acid or benzoic acid. The preservatives will preferably be comprised in the second housing element, i.e. in the water miscible solvent.

Sun protection agents may be selected from p-Aminobenzoic acid, Padimate O, Phenylbenzimidazole sulfonic acid, Cinoxate, Dioxybenzone, Oxybenzone, Homosalate, Menthyl anthranilate, Octocrylene, Octyl methoxycinnamate, Octyl salicylate, Sulisobenzone, Trolamine salicylate, Avobenzone, Ecamsule, Titanium dioxide, Zinc oxide, 4-Methylbenzylidene camphor, Parsol Max, Tinosorb M, Parsol Shield, Tinosorb S, Tinosorb A2B, Neo Heliopan AP, Mexoryl XL, Benzophenone-9, Uvinul T 150, Uvinul A Plus, Uvasorb HEB, Parsol SLX, Amiloxate.

Various other agents may be selected from arginine, cranberry, tea tree oil, xylitol or extracts such as extracts from aloe vera, camomile, milk thistle, walnut, witch hazel, burdock dry, liquorice dry or sage.

Moisture sensitive agents/active water sensible agents may be selected from proteinases/protease enzymes such as papain, bromelain and actinidin. Moisture sensitive agents are defined as compounds that will lose their effect upon contact with water.

### Desiccant

In the present content, "desiccant" is a hygroscopic substance that induces or sustains a state of dryness (desiccation) in its vicinity. Suitable desiccants are selected from one or more of activated charcoal, calcium sulfate (drierite), zeolites, anhydrous calcium chloride, bentonite/clay, calcium oxide, calcium sulfate, molecular sieve and silica gel. Desiccants may be presented in a sachet or porous packet and silica is a well-known type of desiccant that is suitable in the present context. The most preferred desiccant is molecular sieve and/or silica gel. Other preferred desiccants are selected from one or more of anhydrous calcium chloride, bentonite/clay, calcium oxide, calcium sulfate, molecular sieve and silica gel.

In an embodiment of the invention, the desiccant is integrated in the first housing element, such as being and integrated part of the inside walls of the first housing element. Further, the first housing element may be a sachet having a desiccant coating in the inside. In an alternative embodiment, the desiccant is integrated in the first housing element by having an integrated desiccated plastic sleeve. In another embodiment of the invention, a desiccant may be comprised in the first housing element comprising one or more probiotic bacteria or one or more moisture sensitive agents.

### Hydrophobic compounds

In the present context, "hydrophobic compounds" as used herein are compounds that are nonpolar and, thus, prefer other neutral molecules and nonpolar solvents.

Examples of hydrophobic compounds include the alkanes, oils, fats, and greasy substances in general.

### Emulsifiers

In the present context, "emulsifier" is a compound capable of lowering the surface tension (or interfacial tension) between two liquids, or between a liquid and a solid. According to the invention an emulsifier is capable of lowering the surface tension between one or more water miscible solvents of the invention and one or more of hydrophobic compounds of the invention to provide the aqueous topical composition as defined herein. Surfactants may act as detergents, wetting agents, emulsifiers, foaming agents, and dispersants, and they are selected from one or more of alkyl (C₁₂₋₁₅) benzoate, capryl/capramidopropyl betaine, ceteareth-25, Cetomacrogol, emulsifying wax, Cetomacrogol 1000, cetostearyl alcohol, cetylalkohol, cetyl esters wax, cetyl palmitate, cetyl PEG/PPG-10/1 dimethicone, emulsifying wax, ethyl oleate, mono glycerids eg. glycerol monosterate, glycerol monopalmitate, glycerol monooelate, di glycerides eg, glycerol dioelate, glycerol dipalmitate, glycerol disterate, mono- and diglycerids eg. glyceryl behenate, glycerol stearate, glycerol laurate, glycerol palmitate, mono-, di- and triglycerids eg. glyceryl behenate, glycerol stearate, glycerol laurate, glycerol palmitate, hydrogenated cocoglycerides, macrogol cetostearyl ether, macrogol glycerol ricinoleate, macrogol lauryl ether, macrogol oleyl ether, macrogol stearate, macrogol stearyl ether, methyl glucose isostearate, PEG-120 methyl glucose dioleate, PEG-100 stearate, polyglyceryl isosterate, polyglyceryl oleate, polyoxypropylene stearyl ether, polysorbate 20, polysorbate 60, polysorbate 80, propyleneglycol dicaprylate/caprate, ricinoleamidopropyl betaine, sorbitan isostearate, sorbitan laurate, sorbitan oleate, sorbitan sesquilate, sorbitan stearate, sorbitan trioleate, steareth-2, stearyl alcohol, sucrose stearate, sucrose palmitate, sucrose behenate, sucrose myristate, sucrose laurate, sucrose oleate, sucrose erucate, sucrose ester of mixed fatty acids, undecylenamidopropyl betaine, citric acid esters of the mono- and diglycerides, citrilated glycerol monooelate, citrilated glycerol monostearate, lactic acid esters of mono- and diglycerides, high molecular weight copolymers of acrylic acid, C₁₀-C₃₀ alkyl acrylate crosslinked with allyl pentaerythritol, glyceryl behenate, and/or glycerol stearate.

In a preferred embodiment, the emulsifier is selected from one or more of monoglycerides, diglycerides, triglycerides, citric acid esters of mono- and diglycerides, glyceryl behenate/or glycerol stearate and, high molecular weight copolymers of acrylic acid, C₁₀-C₃₀ alkyl acrylate crosslinked with allyl pentaerythritol. In an even more preferred embodiment, said emulsifier is selected from one or more of glyceryl behenate or glycerol stearate.

In a preferred embodiment, the emulsifier is only present in the water miscible solvent.

### Male-female configuration

In a male-female configuration the "female" generally receives and holds the "male". The part bearing one or more protrusions, or fitting inside the other, are designated male, whereas the part containing the corresponding recessions, or fitting outside the other, are designated female.

A male-female configuration is a configuration wherein two mechanical elements, herein the first housing element for holding the viable bacteria of at least one probiotic bacterial strain and the second housing element for holding the water miscible solvent, are fitted together, in a way so that at least a part of one element, e.g. the first housing element have received a part of another element, e.g. the means for holding the hydrophilic phase, such as in a lock/key or a socket/plug configuration. In an embodiment, the male-female configuration is adapted in the form of a stick or a bottle with a cap, preferably a re-attachable cap.

In the present invention, the first housing element may comprise both a female part and a male part. In the same way, the means of holding the hydrophilic phase i.e. the second housing element may also comprise both a female part and a male part. The female part of the means for holding the hydrophilic phase may be complementary to the male part of the first housing element and the male part of the means for holding the hydrophilic phase may be complementary to the female part of the first housing element.

### Snugly fitted configuration

In some embodiments, the male-female configuration of the first housing element and the means for holding the hydrophilic phase i.e. the second housing element comprises a snugly fitted configuration.

A snugly fit is a fit as of mechanical parts with no allowance, e.g. the closest fit that can be assembled for parts that are not meant to move against each other. A snugly fit configuration has the advantage of providing maximum protection against leakage between the surfaces that are in contact with each other.

### Viable in the aqueous topical composition

In the present context, "viable in the aqueous topical composition" is intended to mean that probiotic bacteria remain viable in the aqueous topical composition in the range of 1·10⁶-10¹¹ CFU/g of the viable bacteria of at least one probiotic bacterial strain for at least 5 minutes at 25°C/60%RH or 30°C/75%RH or 5°C, such as for at least 20 minutes at 25°C/60%RH or 30°C/75%RH or 5°C, such as at least 1 hour at 25°C/60%RH or 30°C/75%RH or 5°C, such as at least 4 hours at 25°C/60%RH or 30°C/75%RH or 5°C, such as at least 12 hours at 25°C/60%RH or 30°C/75%RH or 5°C, such as at least 24 hours at 25°C/60%RH or 30°C/75%RH or 5°C, such as at least 2 days at 25°C/60%RH or 30°C/75%RH or 5°C, such as at least 3 days at 25°C/60%RH or 30°C/75%RH or 5°C, such as at least 4 days at 25°C/60%RH or 30°C/75%RH or 5°C, such as at least 5 days at 25°C/60%RH or 30°C/75%RH or 5°C, such as at least 6 days at 25°C/60%RH or 30°C/75%RH or 5°C, such as at least 7 days at 25°C/60%RH or 30°C/75%RH or 5°C, , such as at least 8 days at 25°C/60%RH or 30°C/75%RH or 5°C, such as at least 9 days at 25°C/60%RH or 30°C/75%RH or 5°C, such as at least 10 days at 25°C/60%RH or 30°C/75%RH or 5°C such as at least 10-14 days at 25°C/60%RH or 30°C/75%RH or 5°C

### Percent weight/weight or %(w/w)

In the present context "percent weight/weight" or "%*(w*/*w)*" are used interchangeably and indicates the weight percentage of a component or ingredient as compared to total composition weight, i.e. the weight of the finished composition unless otherwise indicated. In certain cases it may refer to the weight percentage as compared to an intermediate composition.

Unless defined otherwise, all technical and scientific terms used herein have the same meaning as commonly understood by persons skilled in the art. Although any methods and materials equivalent or similar to those described herein can be used in the practice of the present disclosure, typical methods and materials are described. All methods described herein can be performed in any suitable order unless otherwise indicated herein or otherwise clearly contradicted by context.

The use of the terms "a" and "an" and "the" and similar referents in the context of describing the invention (especially in the context of the claims) are to be construed to cover both the singular and the plural, unless otherwise indicated herein or clearly contradicted by context. The terms "comprising", "having", "including" and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

It should be noted that embodiments and features described in the context of one of the aspects of the present invention also apply to the other aspects of the invention.

The invention will now be described in further details in the following non-limiting examples.

### Examples

### Overview of examples

Examples 1.1-1.3: Production and stability of aqueous gels comprising probiotic bacteria.

Example 2: Production and stability of aqueous gel lotion comprising probiotic bacteria.

Example 3.1-3.2: Production and stability of dry compositions comprising probiotic bacteria

Example 4: Stability of commercial product comprising bacteria.

### Materials and methods:

An overview of the excipients and equipment used are shown in Table 1, and the types of probiotic bacteria used are shown in Table 2.

The probiotic bacteria used were *Lactobacillus rhamnosus, Lactobacillus paracasei,* and *Bifidobacterium animalis* subsp *lactis,* Esse Sensitive Serum from Esse skincare was purchased via the internet. Esse Probiotic Serum (Batch 18ADF) from Esse skincare was purchased in a store in Copenhagen.

**Table 1 - Excipients and equipment**

| Brand name | Type | Type | Company |
|---|---|---|---|
| Pemulen^{™} TR-1 NF | Carbomer | high molecular weight crosslinked copolymers of acrylic acid and a hydrophobic C10-30 alkyl acrylate co-monomer | Lubrizol |
| Carbopol Ultrez^{®} 10 NF | Carbomer | Crosslinked Polyacrylic acid copolymer | Lubrizol |
| EMPROVE^{®} exp Ph Eur | Buffer | Sodium Hydrogen carbonate | Merck KGaA |
| Esse Sensitive Serum | Esse Sensitive Serum (Batch | Jojoba seed oil, sesame seed oil, hydrogenated vegetable oil, shea butter, isoamyl laurate, | Esse skincare |
| | 16AXCE, exp. Sep 2018) | lactobacillus, tocopherol, sunflower seed oil, ascorbyl palmitate, vanillin, gamma decalactone. | |
| Esse Probiotic Serum | Esse Probiotic Serum (Batch 18ADF, exp. AUG19) | Jojoba seed oil, sesame seed oil, shea butter, hydrogenated vegetable oil, isoamyl laurate, Marula Seed Oil lactobacillus, tocopherol, sunflower seed oil, ascorbyl palmitate, vanillin, gamma decalactone | Esse skincare |
| Carbopol ETD 2020 NF | Carbomer | Crosslinked Polyacrylic acid copolymer | Lubrizol |
| Tri sodium citrate dihydrate | Buffer | Tri sodium citrate dihydrate | Jungbunzlauer |
| Paraffin oil | Oil | Paraffin oil | Henry Lamotte |
| High Oleic Sunflower Oil | Oil | High Oleic Sunflower Oil | AAK |
| Maximum Recovery Diluent (MRD) Oxoid CM0733 | Maximum Recovery Diluent (MRD) | Maximum Recovery Diluent (MRD) | Oxoid |
| MRS Agar pH 6.2 (Oxoid CM0361) | MRS Agar pH 6.2 | MRS Agar pH 6.2 (Oxoid CM0361) | Oxoid |
| Smasher, bioMérieux | Stomacher | Stomacher (Smasher) | bioMérieux |
| Cysteine, HCl (Merck 102839) | Cysteine, HCl | Cysteine, HCl (Merck 102839) | Merck |
| MColorpHast^{™} | pH-indicator strips 2.0 - 9.0 | | Merck KGaA |

**Table 2 - Strains**

| Type | Brand name | Deposited as No. | Company |
|---|---|---|---|
| *Lactobacillus rhamnosus* | LGG^{®} | ATCC 53103 | Chr. Hansen |
| *Lactobacillus paracasei* | F19^{®} | LMG-P-17806 | Chr. Hansen |
| *Bifidobacterium animalis* subsp *lactis* | BB-12^{®} | DSM 15954 | Chr. Hansen |

### Example 1 - Production and stability of aqueous gel comprising probiotic bacteria

**Table 3 - Composition of three different batches produced**

| Example | 1.1 | 1.2 | 1.3 |
|---|---|---|---|
| Ingredients in mg or g per batch (%, g/100 g gel product) | | | |
| *Lactobacillus rhamnosus* | 4072.9 mg (4.1%) | - | - |
| *Lactobacillus paracasei* | - | 3.55 g (3.5%) | |
| *Bifidobacterium animalis* subsp. *lactis* | - | - | 3.79 g (3.79%) |
| Tri sodium citrate dihydrate | 749.9 mg (0.75%) | 0.75 g (0.75%) | 0.75 g (0.75%) |
| Carbomer (Carbopol Ultrez^{®} 10 NF) | 1500.4 mg (1.5%) | 1.51 g (1.5%) | 1.51 g (1.5%) |
| Demineralized water | 93.64 g (93.7%) | 94.5 g (94.2%) | 93.87 (93.94%) |
| Total weight of ingredients | 99.96 g (100%) | 100.3 g (100%) | 99.92 g (100%) |

### Example 1.1 - Production and stability of aqueous gel comprising Lactobacillus rhamnosus

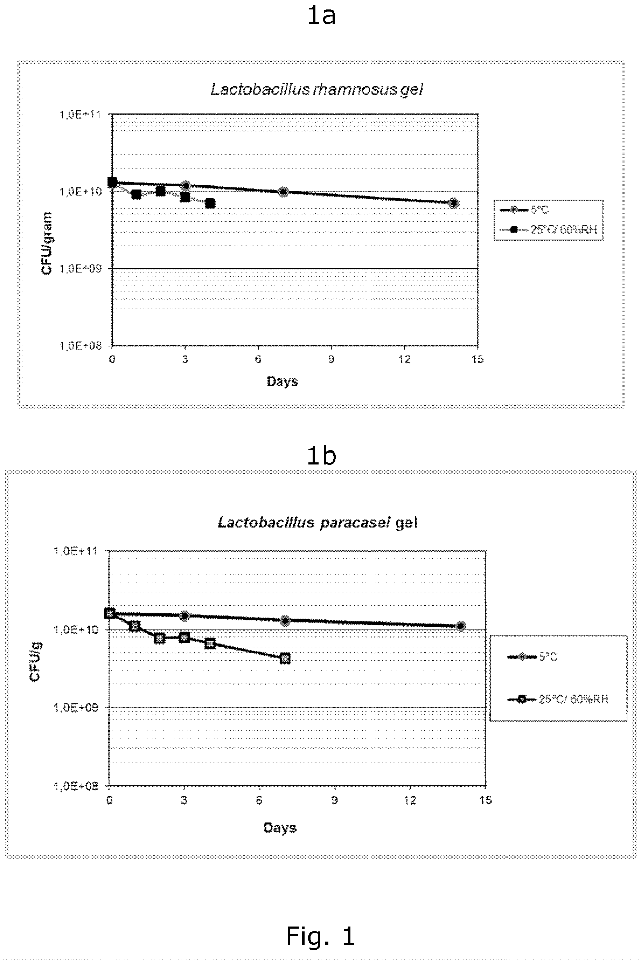

Freeze dried *Lactobacillus rhamnosus* is grinded and afterwards sieved using a 200 µm sieve and stored at -20°C. The powder is acclimatized before weighing the grinded material to be mixed with the liquid phase. Demineralized water is in a 250 ml glass beaker heated to boil and cooled thereafter to room temperature. Carbopol Ultrez^{®} 10 NF is weighed and added slowly to the water under agitation with magnetic stirrer. The mixture is left standing for up to an hour for complete hydration of the carbomer. The pH is measured with strips to be between 2.5 - 3.0. The amount of bacteria and ingredients used for Example 1.1 are found in Table 3. *Lactobacillus rhamnosus* is added under heavily agitation to ensure completely distribution; afterwards tri sodium citrate dihydrate is added under heavily agitation. The full trial batch is immediately portioned into 2 vials with screw lid. One vial is placed at 5°C ± 3°C. At time point 0, 3, 7 and 14 days, the total cell count of *Lactobacillus rhamnosus,* is determined as Colonic Forming Units per g gel product (CFU/g). The other vial is placed at 25°C±3°C/60±5%RH. At time point 0, 1, 2, 3 and 4 days, the total cell count of *Lactobacillus rhamnosus,* is determined as Colonic Forming Units per g gel product (CFU/g). The total viable cell count of *Lactobacillus rhamnosus* is determined as Colony Forming Units per g product (CFU/g) by a pour plate method with MRS Agar pH 6.2 (Oxoid CM0361). Triplicates of 2 g product are mixed with 198 g Maximum Recovery Diluent (MRD) (e.g. Oxoid CM0733) preparing a 1:100 dilution using a Stomacher (e.g. Smasher, bioMérieux) for 2 minutes at 450-550 strokes per minute. A serial dilution in MRD is prepared for each weighing and two dilutions/volumes meeting 30-300 CFU/plate are used (2 x 2 plates for each weighing). The plates are incubated under anaerobic conditions at 37°C for 3 days. The colonies are counted and the result is calculated as the average of the results for the three weighings in CFU/g. The results from the stability setup are found in Table 4 and Figure 1a.

**Table 4 - Comparison of the temperatures and CFU/g values obtained within the stability study of the aqueous gel comprising Lactobacillus rhamnosus.**

| Time, Days | Colony Forming Units per g (CFU/g) | |
|---|---|---|
| | 5°C | 25°C/ 60%RH |
| 0 | 1.3E+10 | 1.3E+10 |
| 1 | NA | 9.1E+09 |
| 2 | NA | 1.0E+10 |
| 3 | 1.2E+10 | 8.4E+09 |
| 4 | NA | 7.1E+09 |
| 7 | 9.9E+09 | - |
| 14 | 7.1E+09 | - |

| | | |
|---|---|---|
| NA: not available | | |

### Conclusions from example 1.1

The results are presented in Figure 1a that shows stability of 4.1% *Lactobacillus rhamnosus* in an aqueous topical composition of the present invention at 5°C (closed circles, upper curve) and at 25°C/60%RH (squared boxes, lower curve). It is evident that the stability of the aqueous topical composition of the present invention comprising *Lactobacillus rhamnosus* at 25°C/60%RH is very good, as an amount of at least 10⁹ CFU/g of said *Lactobacillus rhamnosus* is viable at day 4. It is also evident that the stability of the aqueous topical composition of the present invention comprising *Lactobacillus rhamnosus* at 5°C is even better, as an amount of at least 10⁹ CFU/g of said *Lactobacillus rhamnosus* is viable at day 14. Hence, it has been shown that the aqueous topical composition of the present invention is able to keep the probiotic bacteria *Lactobacillus rhamnosus* viable in a surprisingly high amount and for a surprisingly long time in an aqueous environment i.e. at least 4 days at 25°C/60%RH and at least 14 days at 5°C. The limited decrease in viable cells is surprising and very promising for a commercial product comprising an aqueous topical composition of the invention with a shelf life for at least 14 days at 5°C and for at least 4 days at 25°C/60%RH.

### Example 1.2 - Production and stability of aqueous gel comprising Lactobacillus paracasei

Freeze dried *Lactobacillus paracasei* is grinded and afterwards sieved using a 200 µm sieve and stored at -20°C. The powder is acclimatized before weighing the grinded material to be mixed with the liquid phase. Demineralized water is autoclaved and cooled thereafter to room temperature. The carbomer is weighed into a Blue Cap bottle and water is added to the Blue Cap bottle under agitation with a magnetic stirrer. The mixture is left standing for 2 days for complete hydration of the carbomer. The pH is measured with strips to be between 2.5 - 3.0. The amount of bacteria and ingredients used for Example 1.2 are found in Table 3. *Lactobacillus paracasei* is added under heavily agitation to ensure completely distribution, afterwards tri sodium citrate dihydrate is added under heavily agitation. The full trial batch is immediately portioned into 2 vials with screw lid. One vial is placed at 5°C ± 3°C. At time point 0, 3, 7 and 14 days, the total cell count of *Lactobacillus paracasei,* is determined as Colonic Forming Units per g gel product (CFU/g). The other vial is placed at 25°C±3°C/60±5%RH. At time point 0, 1, 2, 3, 4 and 7 days, the total cell count of *Lactobacillus paracasei,* is determined as Colonic Forming Units per g gel product (CFU/g). The total viable cell count of *Lactobacillus paracasei* is determined as Colony Forming Units per g product (CFU/g) by a pour plate method with MRS Agar pH 6.2 (Oxoid CM0361). Triplicates of 2 g product are mixed with 198 g Maximum Recovery Diluent (MRD) (e.g. Oxoid CM0733) preparing a 1:100 dilution using a Stomacher (e.g. Smasher, bioMérieux) for 2 minutes at 450-550 strokes per minute. A serial dilution in MRD is prepared for each weighing and two dilutions/volumes meeting 30-300 CFU/plate are used (2 x 2 plates for each weighing). The plates are incubated under anaerobic conditions at 37°C for 3 days. The colonies are counted and the result is calculated as the average of the results for the three weighings in CFU/g. The results from the stability setup are found in Table 5 and Figure 1b.

**Table 5 - Comparison of the temperatures and CFU/g values obtained within the stability study of the aqueous gel comprising Lactobacillus paracasei.**

| Time, Days | Colony Forming Units per g (CFU/g) | |
|---|---|---|
| | 5°C | 25°C/ 60%RH |
| 0 | 1.60E+10 | 1.60E+ 10 |
| 1 | NA | 1.10E+10 |
| 2 | NA | 7.70E+09 |
| 3 | 1.50E+10 | 7.9E+09 |
| 4 | NA | 6.6E + 09 |
| 7 | 1.3E+10 | 4.3E+09 |
| 14 | 1.1E + 10 | NA |

| | | |
|---|---|---|
| NA: not available | | |

### Conclusions from example 1.2

The results are presented in Figure 1b that shows stability of 3.5% *Lactobacillus paracasei* in an aqueous topical composition of the present invention at 5°C (closed circles, upper curve) and at 25°C (squared boxes, lower curve). It is evident that the stability of the aqueous topical composition of the present invention comprising *Lactobacillus paracasei* at 25°C/60%RH is very good, as an amount of at least 10⁹ CFU/g of said *Lactobacillus paracasei* is viable at day 7. It is also evident that the stability of the aqueous topical composition of the present invention comprising *Lactobacillus paracasei* at 5°C is even better, as an amount of at least 10¹⁰ CFU/g of said *Lactobacillus paracasei* is viable at day 14. Hence, it has been shown that the aqueous topical composition of the present invention is able to keep the probiotic bacteria *Lactobacillus paracasei* viable in a surprisingly high amount and for a surprisingly long time in an aqueous environment i.e. at least 7 days at 25°C/60%RH or 30°C/75%RH and at least 14 days at 5°C.

### Example 1.3 - Production and stability of aqueous gel comprising Bifidobacterium animalis subsp lactis

Freeze dried *Bifidobacterium animalis* subsp *lactis* is grinded and afterwards sieved using a 200 µm sieve and stored at -20°C. The powder is acclimatized before weighing the grinded material to be mixed with the liquid phase. Tri sodium citrate dihydrate is stored at room temperature and do not need acclimatization before weighing and mixing with the liquid phase. Demineralized water is autoclaved and cooled thereafter to room temperature. The carbomer is weighed into a Blue Cap bottle and water is added to the Blue Cap bottle under agitation with a magnetic stirrer. The mixture is standing for 2 days for complete hydration of the carbomer. The pH is measured with strips to be between 2.5 - 3.0. The amount of bacteria and ingredients used for Example 1.3 are found in Table 3. *Bifidobacterium animalis* subsp *lactis* is added under heavily agitation to ensure completely distribution; afterwards tri sodium citrate dihydrate is added under heavily agitation. The full trial batch is immediately portioned into 2 vials with screw lid. One vial is placed at 5°C ± 3°C. At time point 0, 3, 7 and 14 days, the total cell count of *Bifidobacterium animalis* subsp *lactis,* is determined as Colonic Forming Units per g gel product (CFU/g). The other vial is placed at 25°C±3°C/60±5%RH. At time point 0, 1, 2, 3, 4 and 7 days, the total cell count of *Bifidobacterium animalis* subsp *lactis,* is determined as Colonic Forming Units per g gel product (CFU/g).

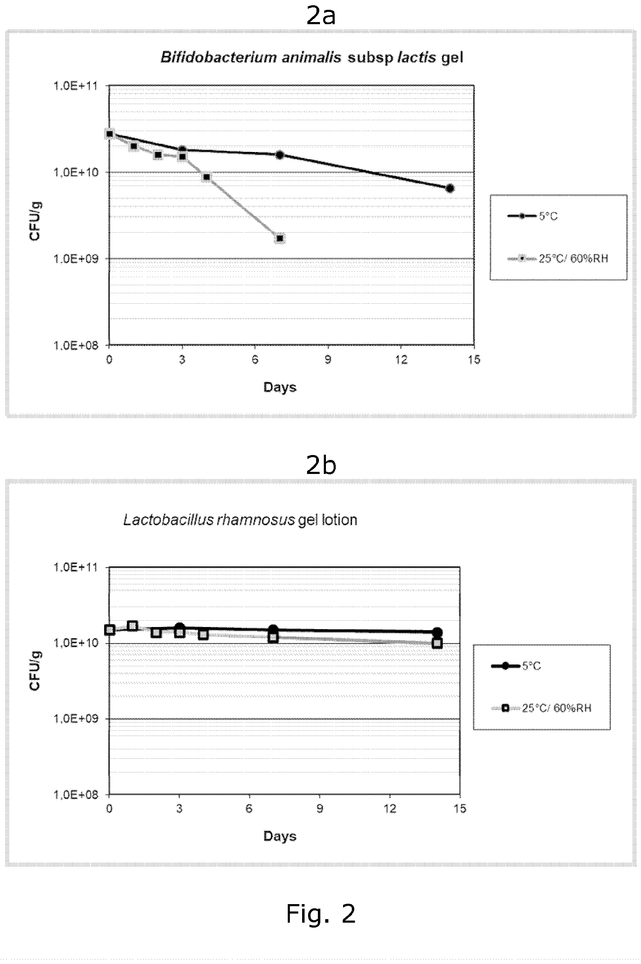

The total viable cell count of *Bifidobacterium animalis* subsp *lactis* is determined as Colony Forming Units per g product (CFU/g) by a pour plate method with MRS Agar pH 6.2 (Oxoid CM0361) with added 0.05% Cysteine, HCl (Merck 102839). Triplicates of 2 g product are mixed with 198 g Maximum Recovery Diluent (MRD) (e.g. Oxoid CM0733) preparing a 1:100 dilution using a Stomacher (e.g. Smasher, bioMérieux) for 2 minutes at 450-550 strokes per minute. A serial dilution in MRD is prepared for each weighing and two dilutions/volumes meeting 30-300 CFU/plate are used (2 × 2 plates for each weighing). The plates are incubated under anaerobic conditions at 37°C for 3 days. The colonies are counted and the result is calculated as the average of the results for the three weighings in CFU/g. The results from the stability setup are found in Table 6 and Figure 2a.

**Table 6 - Comparison of the temperatures and CFU values obtained within the stability study of the aqueous gel comprising Bifidobacterium animalis subsp lactis.**

| Time, Days | Colony Forming Units per g (CFU/g) | |
|---|---|---|
| | 5°C | 25°C/ 60%RH |
| 0 | 2.8E+10 | 2.8E+10 |
| 1 | NA | 2.0E+10 |
| 2 | NA | 1.6E+10 |
| 3 | 1.8E+10 | 1.5E+10 |
| 4 | NA | 8.9E+09 |
| 7 | 1.6E+10 | 1.7E+09 |
| 14 | 6.5E+09 | NA |

| | | |
|---|---|---|
| NA: not available | | |

### Conclusions from example 1.3

The results are presented in Figure 2a and shows stability of 3.79% *Bifidobacterium animalis* subsp *lactis* in an aqueous topical composition of the present invention at 5°C (closed circles, upper curve) and at 25°C (squared boxes, lower curve). It is evident that the stability of the aqueous topical composition of the present invention comprising *Bifidobacterium animalis* subsp *lactis* at 25°C/60%RH is very good, as an amount of at least 10⁹ CFU/g of said *Bifidobacterium animalis* subsp *lactis* is viable at day 7.

It is also evident that the stability of the aqueous topical composition of the present invention comprising *Bifidobacterium animalis* subsp *lactis* at 5°C is even better, as an amount of at least 10⁹ CFU/g of said *Bifidobacterium animalis* subsp *lactis* is viable at day 14.

Hence, it has been shown that the aqueous topical composition of the present invention is able to keep the probiotic bacteria *Bifidobacterium animalis* subsp *lactis* viable in a surprisingly high amount and for a surprisingly long time in an aqueous environment i.e. at least 7 days at 25°C/60%RH or 30°C/75%RH and at least 14 days at 5°C.

### Example 2 - Production and stability of aqueous gel lotion comprising Lactobacillus rhamnosus

**Table 7 - Composition of aqueous gel lotion produced**

| Example | 2 |
|---|---|
| Ingredients in mg or g per batch (%, g/100 g gel lotion product) | |
| *Lactobacillus rhamnosus* | 4073 mg (4.1%) |
| Sodium hydrogen carbonate | 600 mg (0.6%) |
| Carbomer (Carbopol Ultrez^{®} 10 NF) | 0.99 g (1%) |
| Carbomer (Pemulen) | 250 mg (0.25%) |
| Paraffin oil | 15.02 g (15%) |
| Demineralized water | 79.16 g (79%) |
| Total weight of ingredients | 100 g |

A mixture of autoclaved demineralized water at room temperature are mixed with carbomer powder, acryl polymer high molecular weight crosslinked copolymers of acrylic acid and a hydrophobic C10-30 alkyl acrylate co-monomer and paraffin oil, homogenated for 15-20 seconds with homogenizer (increasing speed app. from 3000 rpm up to app. 15000 rpm and back to app. 3000 rpm). The pH is measured with strips to be between 2.5 - 3.0.

The viscosity is measured to app. 200 mPa·s using a shear rate of 2.0 s⁻¹ at 25°C. Freeze dried *Lactobacillus rhamnosus* is weighed and added to the hydrated carbomer lotion and then shaking firmly to ensure proper mixing. The increase in viscosity is seen to some degree within 5-10 seconds upon adding the freeze dried bacteria, sodium hydrogen carbonate is weighed and added to the mixture. The viscosity is within 20-30 seconds increased further.

The viscosity is measured to app. 60000 mPa·s using a shear rate of 2.0 s⁻¹ at 25°C.

The resulting pH is measured with strips to, pH 4.0-4.5. The amount of bacteria and ingredients used for Example 2 are found in Table 7.

The full trial batch is immediately portioned into 2 vials with screw lid. One vial is placed at 5°C ± 3°C. At time point 0, 3, 7 and 14 days, the total cell count of

*Lactobacillus rhamnosus,* is determined as Colonic Forming Units per g gel product (CFU/g). The other vial is placed at 25°C±3°C/60±5%RH. At time point 0, 1, 2, 3, 4, 7 and 14 days, the total cell count of *Lactobacillus rhamnosus,* is determined as Colonic Forming Units per g gel product (CFU/g).

The total viable cell count of *Lactobacillus rhamnosus* is determined as Colony Forming Units per g gel lotion product (CFU/g) by a pour plate method with MRS Agar pH 6.2 (Oxoid CM0361). Triplicates of 2 g product are mixed with 198 g pre-heated (45°C) Maximum Recovery Diluent (MRD) (e.g. Oxoid CM0733) with added 1% Tween 80 preparing a 1:100 dilution using a Stomacher (e.g. Smasher, bioMérieux) for 2 minutes at 450-550 strokes per minute. A serial dilution in MRD is prepared for each weighing and two dilutions/volumes meeting 30-300 CFU/plate are used (2 × 2 plates for each weighing). The plates are incubated under anaerobic conditions at 37°C for 3 days. The colonies are counted and the result is calculated as the average of the results for the three weighings in CFU/g. The results from the stability setup are found in Table 8 and Figure 2b.

**Table 8 - Comparison of the temperatures and CFU values obtained within the stability study of the aqueous lotion containing Lactobacillus rhamnosus**

| Time, Days | Colony Forming Units per g (CFU/g) | |
|---|---|---|
| | 5°C | 25°C/60%RH |
| 0 | 1.5E+10 | 1.5E+10 |
| 1 | N/A | 1.7E+10 |
| 2 | N/A | 1.4E+10 |
| 3 | 1.6E+10 | 1.4E+10 |
| 4 | N/A | 1.3E+10 |
| 7 | 1.5E+10 | 1.2E+10 |
| 14 | 1.4E+10 | 1.0E+10 |

| | | |
|---|---|---|
| NA: not available | | |

### Conclusions from example 2

The results are presented in Figure 2b and Table 8 and show stability of 4.1% *Lactobacillus rhamnosus* in an aqueous topical composition of the present invention at 5°C (closed circles, upper curve) and at 25°C/60%RH (squared boxes, lower curve).

It is evident that the stability of the aqueous topical composition of the present invention comprising *Lactobacillus rhamnosus* at 25°C/60%RH formulated as a gel lotion is surprisingly good, as an amount of at least 1 × 10¹⁰ CFU/g of said *Lactobacillus rhamnosus* is viable at day 14.

It is also evident that the stability of the aqueous topical composition of the present invention comprising *Lactobacillus rhamnosus* at 5°C is equally as good, as an amount of at least 1 × 10¹⁰ CFU/g of said *Lactobacillus rhamnosus* formulated as a gel lotion is viable at day 14.

Hence, it has been shown that the aqueous topical composition of the present invention formulated as a gel lotion is able to keep the probiotic bacteria *Lactobacillus rhamnosus* viable in a surprisingly high amount and for a surprisingly long time in an aqueous lotion environment i.e. at least 14 days at both at 5°C and 25°C/60%RH.

### Example 3 - Production and stability of dry composition comprising bacteria

**Table 9 - Composition of blends (i.e. dry compositions)**

| Example | 3.1 | 3.2 |
|---|---|---|
| Ingredients in mg per product (% product) | | |
| *Lactobacillus rhamnosus* | 22,8 mg (11.4%) | |
| *Bifidobacterium animalis subsp. Lactis* | | 20 mg (10%) |
| Sodium Hydrogen carbonate | 10 mg (5%) | 10 mg (5%) |
| Inulin | 50 mg (25%) | 50 mg (25%) |
| Maltitol | 20 mg (10%) | 20 mg (10%) |
| Mannitol | 97.2 mg (48.6%) | 100 mg (50%) |
| Total weight of ingredients | 200 mg | 200 mg |

### EXAMPLE 3.1 - Stability of Lactobacillus rhamnosus in a first housing element

A blend consisting of *Lactobacillus rhamnosus,* sodium hydrogen carbonate (buffer), inulin, maltitiol and mannitol (fillers) were mixed. The amount of bacteria and ingredients used for Example 3.1 are found in Table 9.

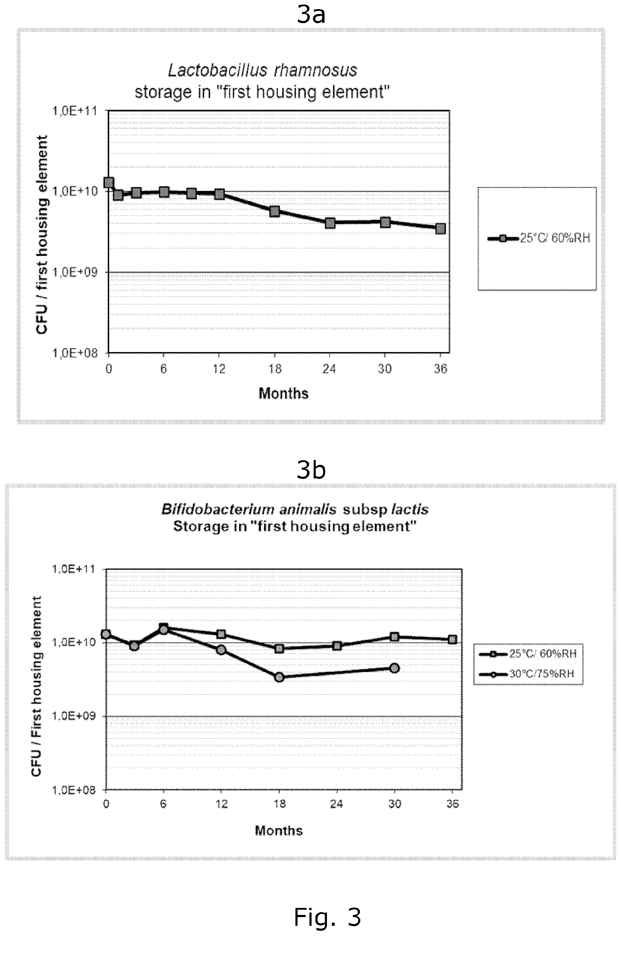

Afterwards 200 mg of the mixture was added to a first housing element for stability trial. The first housing elements were placed at 25°C±3°C/60±5%RH. At time point 0, 1, 3, 6, 9, 12, 18, 24, 30 and 36 months, the total cell count of *Lactobacillus rhamnosus* was determined as Colonic Forming Units per first housing element (CFU/first housing element). The total viable cell count of *Lactobacillus rhamnosus* was determined as Colony Forming Units per first housing element (CFU/first housing element) by a pour plate method with MRS Agar pH 6.2 (Oxoid CM0361). Triplicates of 200 mg product are mixed with app. 200 g Maximum Recovery Diluent (MRD) (e.g. Oxoid CM0733) preparing a 1:1000 dilution using a Stomacher (e.g. Smasher, bioMérieux) for 2 minutes at 450-550 strokes per minute. A serial dilution in MRD is prepared for each weighing and two dilutions/volumes meeting 30-300 CFU/plate are used (2 × 2 plates for each weighing). The plates were incubated under anaerobic conditions at 37°C for 3 days. The colonies are counted and the result is calculated as the average of the results for the three weighings in CFU/first housing element. The results from the stability setup are found in Table 10 and Figure 3a.

**Table 10 - Comparison of the temperatures and CFU values obtained within the stability study of a blend comprising Lactobacillus rhamnosus at 25°C/60%RH or 30°C/75%RH. Comparison of the temperatures and CFU values obtained within the stability study of a blend comprising Bifidobacterium animalis subsp lactis in a first housing element of the invention at 25°C/60%RH or 30°C/75%RH and 30°C/75%RH.**

| Months | Colony Forming Units per g (CFU/g) | | |
|---|---|---|---|
| | 25° C/60%RH | | 30°C±3°C/75±5%RH |
| | *Lactobacillus rhamnosus* | *Bifidobacterium animalis* subsp *lactis* | *Bifidobacterium animalis* subsp *lactis* |
| 0 | 1.3E+10 | 1.3E+10 | 1.3E+10 |
| 1 | 9.0E+09 | NA | NA |
| 3 | 9.6E+09 | 9.1E+09 | 8.9E+09 |
| 6 | 9.9E+09 | 1.6E+10 | 1.5E+10 |
| 9 | 9.5E+09 | NA | NA |
| 12 | 9.3E+09 | 1.3E+10 | 8.0E+09 |
| 18 | 5.7E+09 | 8.3E+09 | 3.4E+09 |
| 24 | 4.1E+09 | 9.0E+09 | NA |
| 30 | 4.2E+09 | 1.2E+10 | 4.5E+09 |
| 36 | 3.5E+09 | 1.1E+10 | NA |

| | | | |
|---|---|---|---|
| NA: not available | | | |

### Conclusions from example 3.1

The results are presented in Figure 3a and Table 10 and show stability of 11.4% *Lactobacillus rhamnosus* in a blend of the present invention at 25°C/60%RH (squared boxes).

It is evident that the stability of the blend in a first housing element of the invention comprising *Lactobacillus rhamnosus* at 25°C/60%RH is surprisingly good, as an amount of at least 1 × 10⁹ CFU/g of said *Lactobacillus rhamnosus* is viable at 36 months.

Hence, it has been shown that the first housing element of the invention is able to keep the probiotic bacteria *Lactobacillus rhamnosus* viable in a surprisingly high amount and for a surprisingly long time in blend comprising *Lactobacillus rhamnosus* i.e. at least 36 months at 25°C/60%RH.

### EXAMPLE 3.2 - Stability of Bifidobacterium animalis subsp lactis in first housing element

A blend consisting of *Bifidobacterium animalis subsp lactis,* sodium hydrogen carbonate (buffer), inulin, maltitiol and mannitol (fillers) were mixed. The amount of bacteria and ingredients used for Example 3.2 are found in Table 9. Afterwards 200 mg of the mixture was added to a first housing element for stability trial. The first housing elements were placed at 25°C±3°C/60±5%RH and 30°C±3°C/75±5%RH. At time point 0, 3, 6, 12, 18, 24, 30 and 36 months (25°C/60%RH) and 0, 3, 6, 12, 18 & 30 months (30°C/75%RH), the total cell count of *Bifidobacterium animalis* subsp *lactis,* was determined as Colonic Forming Units per first housing element (CFU/first housing element). The total viable cell count of *Bifidobacterium animalis* subsp *lactis* was determined as Colony Forming Units per first housing element (CFU/first housing element) by a pour plate method with MRS Agar pH 6.2 (Oxoid CM0361) with added 0.05% Cysteine, HCl (Merck 102839). Triplicates of 200 mg product are mixed with app. 200 g Maximum Recovery Diluent (MRD) (e.g. Oxoid CM0733) preparing a 1:1000 dilution using a Stomacher (e.g. Smasher, bioMérieux) for 2 minutes at 450-550 strokes per minute. A serial dilution in MRD is prepared for each weighing and two dilutions/volumes meeting 30-300 CFU/plate are used (2 × 2 plates for each weighing). The plates were incubated under anaerobic conditions at 37°C for 3 days. The colonies are counted and the result is calculated as the average of the results for the three weighings in CFU/first housing element. The results from the stability setup are found in Table 10 and Figure 3b.

### Conclusions from example 3.2

The results are presented in Figure 3b and Table 10 and show stability of 10.0% *Bifidobacterium animalis* subsp *lactis* in a blend of the present invention at 25°C/60%RH (squared boxes) and at 30°C/75%RH (rounded boxes).

It is evident that the stability of the blend in a first housing element of the invention comprising *Bifidobacterium animalis* subsp *lactis* at 25°C/60%RH is surprisingly good, as an amount of at least 1 × 10¹⁰ CFU/g of said *Bifidobacterium animalis* subsp *lactis* is viable at 36 months.

It is also evident that the stability of the blend in a first housing element of the invention comprising *Bifidobacterium animalis* subsp *lactis* at 30°C/75%RH is surprisingly good, as an amount of at least 3 × 10⁹ CFU/g of said *Bifidobacterium animalis* subsp *lactis* is viable at 30 months.

Hence, it has been shown that the first housing element of the invention is able to keep the probiotic bacteria *Bifidobacterium animalis* subsp *lactis* viable in a surprisingly high amount and for a surprisingly long time in a blend comprising *Bifidobacterium animalis* subsp *lactis* i.e. at least 36 months at 25°C/60%RH and at least 30 months at 30°C/75%RH.

### Example 4 - Determination of viable bacteria cells of Lactobacillus in a commercial product determined at 45°C and 30°C (and aerobic and anaerobic)

Commercial products with claimed viable *Lactobacillus* have also been evaluated with respect to Colony Forming Units per g. The commercial product Esse Sensitive Serum (Batch 16AXCE) from Esse skincare was purchased via the internet and comprises jojoba seed oil, sesame seed oil, hydrogenated vegetable oil, shea butter, isoamyl laurate, lactobacillus, tocopherol, sunflower seed oil, ascorbyl palmitate, vanillin and gamma decalactone. The commercial products have been purchased, stored as recommended at the products until evaluation, and evaluated within the assigned product shelf life.

The total viable cell count of *Lactobacillus* is determined as Colony Forming Units per g product (CFU/g) by a pour plate method with MRS Agar pH 6.2 (Oxoid CM0361). The total viable cell count is determined as Colony Forming Units per g product (CFU/g) by a spread plate method on the surface of Tryptone Soy agar with sheep blood. 1.58 g product are mixed with 160.43 g pre-heated (45°C) Maximum Recovery Diluent (MRD) (e.g. Oxoid CM0733) with added 1% Tween 80 preparing a 1:100 dilution using a Stomacher (e.g. Smasher, bioMérieux) for 2 minutes at 450-550 strokes per minute. A serial dilution in MRD is prepared and two dilutions/volumes meeting 30-300 CFU/plate are used on the 2 plates (3 × 2 × 2 plates). The MRS Agar pH 6.2 plates are incubated under anaerobic conditions at 37°C for 3 days. The Tryptone Soy agar with sheep blood plates are incubated under either aerobic or anaerobic conditions at 37°C for 4 days. The colonies are counted and the result is calculated as CFU/g.

Another commercial product with claimed viable *Lactobacillus* has also been evaluated with respect to Colony Forming Units per g. The commercial product Esse Probiotic Serum (Batch 18ADF) from Esse skincare was purchased in a store in Copenhagen and stored as recommended until evaluation and evaluated within the assigned product shelf life. Esse Probiotic Serum comprises jojoba seed oil, sesame seed oil, hydrogenated vegetable oil, shea butter, isoamyl laurate, marula seed oil, lactobacillus, tocopherol, sunflower seed oil, ascorbyl palmitate, vanillin, gamma decalactone, i.e. the same ingredients as Esse Sensitive Serum except for marula seed oil.

The total viable cell count of *Lactobacillus* is determined as Colony Forming Units per g product (CFU/g) by a pour plate method with MRS Agar pH 6.2 (Oxoid CM0361). 1.58 g product is mixed with 160.43 g pre-heated (30°C) Maximum Recovery Diluent (MRD) (e.g. Oxoid CM0733) with added 1% Tween 80 preparing a 1:100 dilution using a Stomacher (e.g. Smasher, bioMérieux) for 2 minutes at 450-550 strokes per minute. A serial dilution in MRD is prepared and two dilutions/volumes meeting 30-300 CFU/plate are used on the 2 plates (3 × 2 × 2 plates). The MRS Agar pH 6.2 plates are incubated under anaerobic conditions at 37°C for 3 days.

The total viable cell count is also determined as Colony Forming Units per g product (CFU/g) by a spread plate method on the surface of MRS agar.

The product is applied directly on the surface of MRS agar plates and incubated to investigate the presence of any colony forming Lactic acid bacteria.

The product has not been exposed to any sample preparation i.e. heating and dilution. The MRS agar plates are divided into two groups and incubated under aerobic or anaerobic conditions at 37°C for 3 days. The colonies are counted and the result is calculated as CFU/g.

No colonies are found neither on plates incubated under aerobic nor anaerobic conditions.

**Table 11 - Measured level of viable cells of Lactobacillus for commercial products**

| | Pour plate method Colony Forming Units per g (CFU/g) | Spread plate method Colony Forming Units per g (CFU/g) |
|---|---|---|
| Esse Sensitive Serum Batch 16AXCE Tested May 2017 (expiry date of product Sep. 2018) | < 1E+02 CFU/g | < 1E+02 CFU/g |
| | Tested at 45°C | Tested at 45°C |
| Esse Probiotic Serum Batch 18ADF Tested June 2019 (expiry date of product Aug. 2019) | < 1E+02 CFU/g | < 1E+02 CFU/g |
| | Tested at 30°C | Tested at 30°C |

### Conclusions from example 4

The results from the evaluations of viability of *Lactobacillus* are given in Table 11. It is evident that the commercial products do not show any viable *Lactobacillus* at all. Thus, it is evident that the commercial products allegedly comprising viable bacteria cells of *Lactobacillus* are not stable at all, not even from the moment of opening the container comprising the commercial product. It should be noted that the measurements of the commercial products were made well in advance of the expiry date of the product. In addition, it should be noted that using pre-heated (45°C) Maximum Recovery Diluent (MRD) for the pour plate methods have been used in all examples of the present invention, and when testing Esse Sensitive Serum. Additional tests have been made using pre-heated (30°C) Maximum Recovery Diluent (MRD) showing *no difference* i.e. Esse Sensitive Serum and Esse Probiotic Serum comprise no live bacteria i.e. < 1E+02 CFU/g *Lactobacillus.*

## Claims

1. A method of preparing an aqueous topical composition comprising
a) providing viable bacteria of at least one probiotic bacterial strain in an amount of at least 5·10⁶ CFU/g and at least one buffer,
b) providing a mixture comprising at least one thickening agent selected from one or more of carbomer, poloxamer, PEG/macrogol and/or carboxylic acid and at least 60%(w/w) water, and
c) adding said viable bacteria of at least one probiotic bacterial strain of step a) to said mixture of step b)
to prepare said aqueous topical composition having a pH of 4.0-6.7 and a viscosity of more than 2500 mPa·s and an amount of 1·10⁶-10¹¹ CFU/g of said viable bacteria of at least one probiotic bacterial strain, and wherein said viable bacteria of at least one probiotic bacterial strain are viable in the aqueous topical composition for up to 7 days at 25°C/60%RH.

2. The method of preparing an aqueous topical composition according to claim 1 comprising
a) providing a dry composition of viable bacteria of at least one probiotic bacterial strain in an amount of at least 5·10⁶ CFU/g and at least one buffer, wherein said dry composition of viable bacteria of at least one probiotic bacterial strain is powdered having a water activity of no more than 0.30 and a particle size distribution below 500µm
b) providing a mixture comprising at least one thickening agent selected from one or more of carbomer, poloxamer, PEG/macrogol and/or carboxylic acid and water in an amount above 60%(w/w) to provide said mixture having a pH of 2-3 and a viscosity of less than 1000 mPa·s, and
c) adding said dry composition of step a) to said mixture of step b)
to prepare said aqueous topical composition having a pH of 4.0-6.7 and a viscosity of more than 2500 mPa·s and an amount in the range of 1·10⁶-10¹¹ CFU/g of said viable bacteria of at least one probiotic bacterial strain and,
wherein said viable bacteria of at least one probiotic bacterial strain are viable in the aqueous topical composition for up to 7 days at 25°C/60%RH.

3. The method of preparing an aqueous topical composition according to any one of claims 1 or 2, wherein said at least one thickening agent is one or more of carbomers and wherein said dry composition comprises at least one buffer that is selected from one or more of monovalent cations.

4. The method of preparing an aqueous topical composition according to claim 1 comprising
a) providing a dry composition of viable bacteria of at least one probiotic bacterial strain in an amount of at least 5·10⁶ CFU/g and at least one buffer, wherein said dry composition of viable bacteria of at least one probiotic bacterial strain is powdered having a water activity of no more than 0.30 and a particle size distribution below 500µm
b) providing a mixture comprising at least one thickening agent selected from one or more of poloxamers and at least 60%(w/w) water to provide said mixture having a viscosity of about 100 mPa·s when the temperature is no more than 10°C, and a viscosity of about 1000 mPa·s when the temperature is above 25°C, and
c) adding said dry composition of step a) to said mixture of step b)
to prepare said aqueous topical composition having a viscosity of less than 500 mPa·s when the temperature is no more than 10°C, and a viscosity of more than 2500 mPa·s when the temperature is above 30°C, and wherein said aqueous topical composition comprises an amount in the range of 1·10⁶-10¹¹ CFU/g of said viable bacteria of at least one probiotic bacterial strain and wherein said viable bacteria of at least one probiotic bacterial strain are viable in the aqueous topical composition for up to 7 days at 25°C/60%RH.

5. An aqueous topical composition comprising at least 60%(w/w) water and
- viable bacteria of at least one probiotic bacterial strain,
wherein an amount of 1·10⁶-10¹¹ CFU/g of said viable bacteria of at least one probiotic bacterial strain is viable in the aqueous topical composition for up to 7 days at 25°C/60%RH, and wherein said composition comprises at least one thickening agent selected from one or more of carbomer, poloxamer, PEG/macrogol and/or carboxylic acid, and at least one buffer.

6. The aqueous topical composition according to claim 5, wherein said composition comprises at least one carbomer and at least one buffer or at least one poloxamer or a combination thereof.

7. A probiotic bacteria delivery device comprising
- a first housing element comprising viable bacteria of at least one probiotic bacterial strain and at least one buffer and optionally a desiccant, and
- a second housing element comprising at least one thickening agent selected from one or more of carbomer, poloxamer, PEG/macrogol and/or carboxylic acid, and at least 60% (w/w) water, and at least one buffer,
wherein the amount of viable bacteria of said at least one probiotic bacterial strain in said first housing element is at least 5·10⁶ CFU/g for up to 3 years at 25°C/60%RH.

8. The delivery device according to claim 8, wherein said first and second housing elements are separate elements.

9. The delivery device according to claim 7 or 8, wherein said first housing element and said second housing element is each separately sealed with a lid, and wherein said housing elements and said lids provide storage of said viable bacteria of at least one probiotic bacterial strain and said water miscible solvent, without any leakage of said viable bacteria of at least one probiotic bacterial strain and/or leakage of said water miscible solvent during storage.

10. A method of preparing a delivery device according to any of claims 7 to 9 comprising the steps of
a) providing in a first housing element viable bacteria of at least one probiotic bacterial strain in an amount of at least 5·10⁶ CFU/g for up to 3 years at 25°C/60%RH, and
b) providing in a second housing element at least one thickening agent selected from one or more of carbomer, poloxamer, PEG/macrogol and/or carboxylic acid and at least 60% (w/w) water, and at least one buffer
wherein a) and b) together provides said delivery device.

11. Use of a delivery device according to any one of claims 7 to 9 for providing viable bacteria of at least one probiotic bacterial strain in an aqueous topical composition according to claim 5 or 6 for at for at least 7 days at 25°C/60%RH or 30°C/75%RH.

## Patentansprüche

1. Verfahren zum Herstellen einer wässrigen topischen Zusammensetzung, umfassend
a) Bereitstellen lebensfähiger Bakterien mindestens eines probiotischen Bakterienstamms in einer Menge von mindestens 5·10⁶ KBE/g und mindestens einem Puffer,
b) Bereitstellen einer Mischung, die mindestens ein Verdickungsmittel, ausgewählt aus einem oder mehreren von Carbomer, Poloxamer, PEG/Macrogol und/oder Carbonsäure, und mindestens 60 % (Gew.-%) Wasser umfasst, und
c) Hinzufügen der lebensfähigen Bakterien mindestens eines probiotischen Bakterienstamms aus Schritt a) zu der Mischung aus Schritt b),
um die wässrige topische Zusammensetzung mit einem pH-Wert von 4,0-6,7 und einer Viskosität von mehr als 2500 mPa·s und einer Menge von 1·10⁶-10¹¹ KBE/g der lebensfähigen Bakterien mindestens eines probiotischen Bakterienstamms herzustellen, wobei die lebensfähigen Bakterien mindestens eines probiotischen Bakterienstamms in der wässrigen topischen Zusammensetzung bis zu 7 Tage bei 25 °C/60 % RH lebensfähig sind.

2. Verfahren zum Herstellen einer wässrigen topischen Zusammensetzung nach Anspruch 1, umfassend
a) Bereitstellen einer trockenen Zusammensetzung lebensfähiger Bakterien mindestens eines probiotischen Bakterienstamms in einer Menge von mindestens 5·10⁶ KBE/g und mindestens eines Puffers, wobei die trockene Zusammensetzung lebensfähiger Bakterien mindestens eines probiotischen Bakterienstamms pulverisiert ist und eine Wasseraktivität von nicht mehr als 0,30 und eine Teilchengrößenverteilung unter 500 um aufweist
b) Bereitstellen einer Mischung, die mindestens ein Verdickungsmittel, ausgewählt aus einem oder mehreren von Carbomer, Poloxamer, PEG/Macrogol und/oder Carbonsäure, und Wasser in einer Menge von mehr als 60 % (Gew.-%) umfasst, um die Mischung mit einem pH-Wert von 2-3 und einer Viskosität von weniger als 1000 mPa·s bereitzustellen, und
c) Hinzufügen der lebensfähigen Bakterien mindestens eines probiotischen Bakterienstamms aus Schritt a) zu der Mischung aus Schritt b), um die wässrige topische Zusammensetzung mit einem pH-Wert von 4,0-6,7 und einer Viskosität von mehr als 2500 mPa·s und einer Menge von 1·10⁶-10¹¹ KBE/g der lebensfähigen Bakterien mindestens eines probiotischen Bakterienstamms herzustellen, und
wobei die lebensfähigen Bakterien mindestens eines probiotischen Bakterienstamms in der wässrigen topischen Zusammensetzung bis zu 7 Tage bei 25 °C/60 % RH lebensfähig sind.

3. Verfahren zum Herstellen einer wässrigen topischen Zusammensetzung nach einem der Ansprüche 1 oder 2, wobei das mindestens eine Verdickungsmittel ein oder mehrere Carbomere ist und wobei die trockene Zusammensetzung mindestens einen Puffer umfasst, der aus einem oder mehreren einwertigen Kationen ausgewählt ist.

4. Verfahren zum Herstellen einer wässrigen topischen Zusammensetzung nach Anspruch 1, umfassend
a) Bereitstellen einer trockenen Zusammensetzung lebensfähiger Bakterien mindestens eines probiotischen Bakterienstamms in einer Menge von mindestens 5·10⁶ KBE/g und mindestens eines Puffers, wobei die trockene Zusammensetzung lebensfähiger Bakterien mindestens eines probiotischen Bakterienstamms pulverisiert ist und eine Wasseraktivität von nicht mehr als 0,30 und eine Teilchengrößenverteilung unter 500 um aufweist
b) Bereitstellen einer Mischung, die mindestens ein Verdickungsmittel, ausgewählt aus einem oder mehreren Poloxameren, und mindestens 60 % (Gew.-%) Wasser umfasst, um die Mischung mit einer Viskosität von etwa 100 mPa·s, wenn die Temperatur nicht mehr als 10 °C beträgt, und einer Viskosität von etwa 1000 mPa·s, wenn die Temperatur über 25 °C liegt, bereitzustellen, und
c) Hinzufügen der trockenen Zusammensetzung aus Schritt a) zu der Mischung aus Schritt b), um die wässrige topische Zusammensetzung herzustellen, die eine Viskosität von weniger als 500 mPa·s aufweist, wenn die Temperatur nicht mehr als 10 °C beträgt, und eine Viskosität von mehr als 2500 mPa·s, wenn die Temperatur über 30 °C beträgt, und wobei die wässrige topische Zusammensetzung eine Menge im Bereich von 1·10⁶-10¹¹ KBE/g der lebensfähigen Bakterien mindestens eines probiotischen Bakterienstamms umfasst und wobei die lebensfähigen Bakterien mindestens eines probiotischen Bakterienstamms in der wässrigen topischen Zusammensetzung für bis zu 7 Tage bei 25 °C/60 % RH lebensfähig sind.

5. Wässrige topische Zusammensetzung, umfassend mindestens 60 % (Gew.-%) Wasser und
- lebensfähige Bakterien mindestens eines probiotischen Bakterienstamms,
wobei eine Menge von 1·10⁶-10¹¹ KBE/g der lebensfähigen Bakterien mindestens eines probiotischen Bakterienstammes in der wässrigen topischen Zusammensetzung für bis zu 7 Tage bei 25 °C/60 % RH lebensfähig ist, und wobei die Zusammensetzung mindestens ein Verdickungsmittel, ausgewählt aus einem oder mehreren von Carbomer, Poloxamer, PEG/Macrogol und/oder Carbonsäure, und mindestens einen Puffer umfasst.

6. Wässrige topische Zusammensetzung nach Anspruch 5, wobei die Zusammensetzung mindestens ein Carbomer und mindestens einen Puffer oder mindestens ein Poloxamer oder eine Kombination davon umfasst.

7. Vorrichtung zur Abgabe probiotischer Bakterien, umfassend
- ein erstes Gehäuseelement, das lebensfähige Bakterien mindestens eines probiotischen Bakterienstamms und mindestens einen Puffer und optional ein Trockenmittel umfasst, und
- ein zweites Gehäuseelement, umfassend mindestens ein Verdickungsmittel, ausgewählt aus einem oder mehreren von Carbomer, Poloxamer, PEG/Macrogol und/oder Carbonsäure, und mindestens 60 % (Gew.-%) Wasser, und mindestens einen Puffer,
wobei die Menge an lebensfähigen Bakterien des mindestens einen probiotischen Bakterienstammes in dem ersten Gehäuseelement mindestens 5·10⁶ KBE/g für bis zu 3 Jahre bei 25 °C/60 % RH beträgt.

8. Abgabevorrichtung nach Anspruch 8, wobei das erste und das zweite Gehäuseelement getrennte Elemente sind.

9. Abgabevorrichtung nach Anspruch 7 oder 8, wobei das erste Gehäuseelement und das zweite Gehäuseelement jeweils separat mit einem Deckel verschlossen sind, und wobei die Gehäuseelemente und die Deckel eine Speicherung der lebensfähigen Bakterien mindestens eines probiotischen Bakterienstamms und des mit Wasser mischbaren Lösungsmittels bereitstellen, ohne dass es zu einem Auslaufen der lebensfähigen Bakterien mindestens eines probiotischen Bakterienstamms und/oder einem Auslaufen des mit Wasser mischbaren Lösungsmittels während der Speicherung kommt.

10. Verfahren zur Herstellung einer Abgabevorrichtung nach einem der Ansprüche 7 bis 9, umfassend die folgenden Schritte
a) Bereitstellen von lebensfähigen Bakterien mindestens eines probiotischen Bakterienstammes in einem ersten Gehäuseelement in einer Menge von mindestens 5·10⁶ KBE/g für bis zu 3 Jahre bei 25 °C/60 % RH und
b) Bereitstellen in einem zweiten Gehäuseelement mindestens eines Verdickungsmittels, ausgewählt aus einem oder mehreren von Carbomer, Poloxamer, PEG/Macrogol und/oder Carbonsäure und mindestens 60 % (Gew.-%) Wasser, und mindestens eines Puffers wobei a) und b) zusammen die Abgabevorrichtung bereitstellen.

11. Verwendung einer Abgabevorrichtung nach einem der Ansprüche 7 bis 9 zum Bereitstellen lebensfähiger Bakterien mindestens eines probiotischen Bakterienstammes in einer wässrigen topischen Zusammensetzung nach Anspruch 5 oder 6 für mindestens 7 Tage bei 25 °C/60 % RH oder 30 °C/75 % RH.

## Revendications

1. Procédé de préparation d'une composition topique aqueuse comprenant
a) la fourniture de bactéries viables d'au moins une souche bactérienne probiotique en une quantité d'au moins 5·10⁶ CFU/g et au moins un tampon,
b) la fourniture d'un mélange comprenant au moins un agent épaississant choisi parmi un ou plusieurs d'un carbomère, d'un poloxamère, d'un PEG/macrogol et/ou d'un acide carboxylique et au moins 60 % (p/p) d'eau et
c) l'ajout desdites bactéries viables d'au moins une souche bactérienne probiotique de l'étape a) audit mélange de l'étape b)
pour préparer ladite composition topique aqueuse ayant un pH de 4,0 à 6,7 et une viscosité supérieure à 2 500 mPa-s et une quantité de 1,10⁶ à 10¹¹ CFU/g desdites bactéries viables d'au moins une souche bactérienne probiotique, et dans lequel lesdites bactéries viables d'au moins une souche bactérienne probiotique sont viables dans la composition topique aqueuse jusqu'à 7 jours à 25 °C/60 % d'humidité relative.

2. Procédé de préparation d'une composition topique aqueuse selon la revendication 1 comprenant
a) la fourniture d'une composition sèche de bactéries viables d'au moins une souche bactérienne probiotique en une quantité d'au moins 5·10⁶ CFU/g et au moins un tampon, dans lequel ladite composition sèche de bactéries viables d'au moins une souche bactérienne probiotique est en poudre ayant une activité d'eau ne dépassant pas 0,30 et une distribution granulométrique inférieure à 500 um
b) la fourniture d'un mélange comprenant au moins un agent épaississant choisi parmi un ou plusieurs d'un carbomère, d'un poloxamère, d'un PEG/macrogol et/ou d'un acide carboxylique et de l'eau en une quantité supérieure à 60 % (p/p) pour fournir ledit mélange ayant un pH de 2 à 3 et une viscosité inférieure à 1 000 mPa·s, et
c) l'ajout de ladite composition sèche de l'étape a) audit mélange de l'étape b) pour préparer ladite composition topique aqueuse ayant un pH de 4,0 à 6,7 et une viscosité supérieure à 2 500 mPa·s et une quantité dans la plage de 1·10⁶ à 10¹¹ CFU/g desdites bactéries viables d'au moins une souche bactérienne probiotique et,
dans lequel lesdites bactéries viables d'au moins une souche bactérienne probiotique sont viables dans la composition topique aqueuse jusqu'à 7 jours à 25 °C/60 % d'humidité relative.

3. Procédé de préparation d'une composition topique aqueuse selon l'une quelconque des revendications 1 ou 2, dans lequel ledit au moins un agent épaississant est un ou plusieurs parmi des carbomères et dans lequel ladite composition sèche comprend au moins un tampon qui est choisi parmi un ou plusieurs parmi des cations monovalents.

4. Procédé de préparation d'une composition topique aqueuse selon la revendication 1 comprenant
a) la fourniture d'une composition sèche de bactéries viables d'au moins une souche bactérienne probiotique en une quantité d'au moins 5·10⁶ CFU/g et au moins un tampon, dans lequel ladite composition sèche de bactéries viables d'au moins une souche bactérienne probiotique est en poudre ayant une activité d'eau ne dépassant pas 0,30 et une distribution granulométrique inférieure à 500 um
b) la fourniture d'un mélange comprenant au moins un agent épaississant choisi parmi un ou plusieurs poloxamères et au moins 60 % (p/p) d'eau pour fournir ledit mélange ayant une viscosité d'environ 100 mPa·s lorsque la température n'est pas supérieure à 10 °C, et une viscosité d'environ 1 000 mPa·s lorsque la température est supérieure à 25 °C, et
c) l'ajout de ladite composition sèche de l'étape a) audit mélange de l'étape b) pour préparer ladite composition topique aqueuse ayant une viscosité inférieure à 500 mPa·s lorsque la température n'est pas supérieure à 10 °C, et une viscosité supérieure à 2 500 mPa·s lorsque la température est supérieure à 30 °C, et dans lequel ladite composition topique aqueuse comprend une quantité dans la plage de 1·10⁶ à 10¹¹ CFU/g desdites bactéries viables d'au moins une souche bactérienne probiotique et dans lequel lesdites bactéries viables d'au moins une souche bactérienne probiotique sont viables dans la composition topique aqueuse jusqu'à 7 jours à 25 °C/60 % d'humidité relative.

5. Composition topique aqueuse comprenant au moins 60 % (p/p) d'eau et
- des bactéries viables d'au moins une souche bactérienne probiotique,
dans lequel une quantité de 1·10⁶ à 10¹¹ CFU/g desdites bactéries viables d'au moins une souche bactérienne probiotique est viable dans la composition topique aqueuse jusqu'à 7 jours à 25 °C/60 % d'humidité relative, et dans lequel ladite composition comprend au moins un agent épaississant choisi parmi un ou plusieurs d'un carbomère, d'un poloxamère, d'un PEG/macrogol et/ou d'un acide carboxylique, et au moins un tampon.

6. Composition topique aqueuse selon la revendication 5, dans laquelle ladite composition comprend au moins un carbomère et au moins un tampon ou au moins un poloxamère ou une combinaison de ceux-ci.

7. Dispositif d'administration de bactéries probiotiques comprenant
- un premier élément de boîtier comprenant des bactéries viables d'au moins une souche bactérienne probiotique et au moins un tampon et éventuellement un déshydratant, et
- un second élément de boîtier comprenant au moins un agent épaississant choisi parmi un ou plusieurs d'un carbomère, d'un poloxamère, d'un PEG/macrogol et/ou d'un acide carboxylique, et au moins 60 % (p/p) d'eau, et au moins un tampon,
dans lequel la quantité de bactéries viables de ladite au moins une souche bactérienne probiotique dans ledit premier élément de boîtier est d'au moins 5·10⁶ CFU/g jusqu'à 3 ans à 25 °C/60 % d'humidité relative.

8. Dispositif d'administration selon la revendication 8, dans lequel lesdits premier et second éléments de boîtier sont des éléments séparés.

9. Dispositif d'administration selon la revendication 7 ou 8, dans lequel ledit premier élément de boîtier et ledit second élément de boîtier sont chacun scellés séparément avec un couvercle, et dans lequel lesdits éléments de boîtier et lesdits couvercles assurent le stockage desdites bactéries viables d'au moins une souche bactérienne probiotique et ledit solvant miscible à l'eau, sans aucune fuite desdites bactéries viables d'au moins une souche bactérienne probiotique et/ou fuite dudit solvant miscible à l'eau pendant le stockage.

10. Procédé de préparation d'un dispositif d'administration selon l'une quelconque des revendications 7 à 9 comprenant les étapes
a) de fourniture dans un premier élément de boîtier des bactéries viables d'au moins une souche bactérienne probiotique en une quantité d'au moins 5·10⁶ CFU/g jusqu'à 3 ans à 25 °C/60 % d'humidité relative, et
b) de fourniture d'un second élément de boîtier d'au moins un agent épaississant choisi parmi un ou plusieurs d'un carbomère, d'un poloxamère, d'un PEG/macrogol et/ou d'un acide carboxylique, et au moins 60 % (p/p) d'eau, et au moins un tampon,
dans lequel a) et b) fournissent ensemble ledit dispositif d'administration.

11. Utilisation d'un dispositif d'administration selon l'une quelconque des revendications 7 à 9 pour fournir des bactéries viables d'au moins une souche bactérienne probiotique dans une composition topique aqueuse selon la revendication 5 ou 6 pendant au moins 7 jours à 25 °C/60 % d'humidité relative ou 30 °C/75 % d'humidité relative.
